(19) Europäisches Patentamt
European Patent Office
Office européen des brevets

(11) Veröffentlichungsnummer: **0 014 815**
**A2**

(12) # EUROPÄISCHE PATENTANMELDUNG

(21) Anmeldenummer: **79810184.6**

(22) Anmeldetag: **18.12.79**

(51) Int. Cl.³: **C 07 C 103/52**
A 61 K 37/02

(30) Priorität: **20.12.78 CH 12942/78**

(43) Veröffentlichungstag der Anmeldung:
**03.09.80 Patentblatt 80/18**

(84) Benannte Vertragsstaaten:
**AT BE CH DE FR GB IT NL SE**

(71) Anmelder: **CIBA-GEIGY AG**
**Patentabteilung Postfach**
**CH-4002 Basel(CH)**

(72) Erfinder: **Tarcsay, Lajos, Dr.**
**Muttenzerstrasse 25**
**D-7889 Grenzach-Wyhlen(DE)**

(72) Erfinder: **Kamber, Bruno, Dr.**
**Sonnenweg 9**
**CH-4144 Arlesheim(CH)**

(72) Erfinder: **Stanek, Jaroslav, Dr.**
**Florastrasse 6**
**CH-4127 Birsfelden(CH)**

(72) Erfinder: **Baschang, Gerhard, Dr.**
**Bückenweg 7**
**CH-4126 Bettingen(CH)**

(72) Erfinder: **Hartmann, Albert, Dr.**
**Steingasse 21A**
**D-7889 Grenzach(DE)**

(54) **Peptidderivate, Verfahren zu deren Herstellung und Zwischenprodukte sowie pharmazeutische Präparate mit einer dieser Verbindungen.**

(57) Die Erfindung betrifft Lipopeptide der Formel

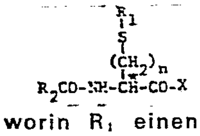

(I)

n = 1,2

* = S/R oder S oder R

worin $R_1$ einen aliphatischen oder aliphatisch-cycloaliphatischen Kohlenwasserstoffrest mit mindestens 9 C-Atomen oder einen aliphatischen Acylrest mit 12-21 C-Atomen oder den 5-Cholesten-3-yl-Rest bedeutet, wobei im genannten Kohlenwasserstoff- oder Acylrest die C-Atom-Kette auch durch Sauerstoffgruppen unterbrochen sein kann, und/oder worin als Substituenten maximal eine freie oder mit einer monobasischen Carbonsäure veresterte Hydroxylgruppe vorhanden sein können, und X eine peptidisch gebundene freie, veresterte oder amidierte Aminosäure oder eine Aminosäurensequenz von 2-10 Aminosäuren, deren terminale Carboxylgruppe in freier, veresterter oder amidierter Form vorliegt, bedeutet, und Diastereomerengemische dieser Verbindungen, Salze und Komplexe.

Die neuen Verbindungen besitzen eine ausgesprochene immunpotenzierende Wirkung. Sie können synthetisch, z.B. durch Einführung der Gruppe X der obigen Formel in entsprechende Carbonsäuren nach üblichen Methoden der Peptidchemie hergestellt werden.

EP 0 014 815 A2

Croydon Printing Company Ltd.

BEZEICHNUNG GEÄNDERT
siehe Titelseite

## Neue Peptidderivate

Die vorliegende Erfindung betrifft neue Lipopeptide und insbesondere Verbindungen der Formel

$R_1$
|
S
|
$(CH_2)_n$
| *
$R_2CO\text{-}NH\text{-}CH\text{-}CO\text{-}X$ (I)

$n = 1,2$
$* = S/R$ oder S oder R

worin $R_1$ einen unsubstituierten oder höchstens an einem der dem Schwefelatom nicht benachbarten C-Atome durch eine freie oder eine mit einer einbasischen Carbonsäure veresterte Hydroxygruppe substituierten und gegebenenfalls durch eine oder mehrere Sauerstoffatome in der C-Atom-Kette unterbrochenen, gesättigten oder ungesättigten aliphatischen Kohlenwasserstoffrest mit mindestens 9 C-Atomen, der auch durch maximal 2 cycloaliphatische Kohlenwasserstoffreste mit 5-8 Ring-C-Atomen substituiert sein kann, oder den Rest $-CO-R_1'$, worin $R_1'$ einen gesättigten oder ungesättigten, aliphatischen oder gemischt aliphatisch-cycloaliphatischen, gegebenenfalls auch durch Sauerstoffatome in der C-Atom-Kette unterbrochenen Kohlenwasserstoffrest mit 11-21 C-Atomen, bedeutet, oder 5-Cholesten-3-yl darstellt, $R_2$ einen gesättigten oder ungesättigten, aliphatischen oder gemischt aliphatisch-cycloaliphatischen, gegebenenfalls auch durch Sauerstoffunktionen substituierten Kohlenwasserstoffrest mit 11 - 21 C-Atomen, und X eine peptidisch gebundene Aminosäure mit freier, veresterter oder amidierter Carboxylgruppe, oder eine Aminosäurensequenz von 2 - 10 Aminosäuren, deren terminale Carboxylgruppe in freier, veresterter oder amidierter Form vorliegt, bedeuten, wobei das mit * bezeichnete Asymmetrie-Zentrum die absolute S- oder R-Konfiguration besitzt, und gegebenenfalls Diastereomerengemische solcher Verbindungen, sowie Salze und Komplexe, sowie Verfahren zur synthetischen Herstellung dieser Verbindungen bzw. Gemische und pharmazeutische Präparate enthaltend einen oder mehrere dieser Lipopeptide zusammen mit einem pharmazeutischen Trägermaterial und gegebenenfalls zusammen mit anderen pharmazeutischen Verbindungen.

Lipopeptide sind als Abbau-Fragmente von Lipoproteinen bereits beschrieben worden. So haben z.B. Hantke und Braun (Eur. J. Biochem. 34, 284 - 296 (1973), aus dem Murein-Lipoprotein der äusseren Zellwand von Escherichia coli durch enzymatische Degradation Lipopeptide bzw. Lipopeptidgemische in unreiner Form isolieren können, denen gemäss ihren Untersuchungen die folgende Struktur

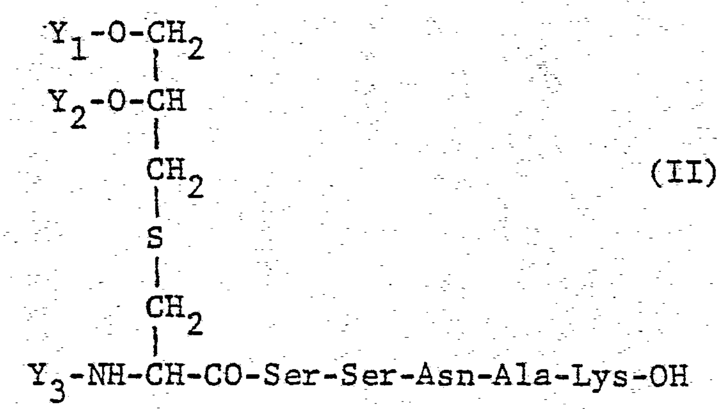

(II)

oder entsprechende Formeln mit verkürzter Polypeptid-Kette zukommen dürfte, wobei $Y_1$, $Y_2$, $Y_3$ Acylreste verschiedener höherer gesättigter und ungesättigter Fettsäuren, wie etwa solcher mit 14-19 C-Atomen und anderer, darstellen. Dieselbe Struktur liegt auch dem Murein-Protein als solchem zugrunde (loc.cit.), wobei die absolute Konfiguration an den beiden asymmetrischen Kohlenstoffatomen der obigen Formel nicht untersucht wurde. Sowohl das Murein-Lipoprotein wie seine genannten Abbauprodukte zeigen in vitro eine mitogene Wirkung gegenüber Mäuse-Lymphocyten. [Vgl. auch Z. Immun.-Forschung, Vol. 153, pp. 11-22 (1977)].

Wie ersichtlich, liegt den aus Mureinprotein erhaltenen Abbau-Lipopeptiden das "Glycerylcystein"

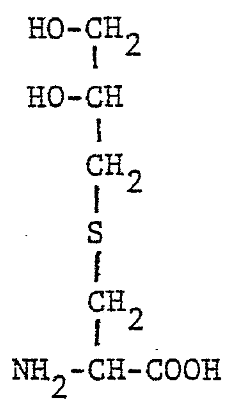

(III)

zugrunde. Als Produkte einer enzymatischen Spaltung von Naturprodukten waren diese Fragmente keine in ihrer Zusammensetzung wohl definierten Produkte und stellten offensichtlich komplizierte Gemische von Kondensationsprodukten aus dem genannten Peptidanteil und sehr verschiedenen Acylderivaten des Glyzerylcysteins dar.

Die Lipopeptide der vorliegenden Anmeldung unterscheiden sich nun von den genannten Abbauprodukten von Murein-Proteinen in mancherlei Hinsicht: sie stellen Lipopeptide von genau definierter einheitlicher chemischer Konstitution und Konfiguration dar, die synthetisch zugänglich sind und sich daher für eine therapeutische Anwendung eignen. Anstelle des veresterten Glyzerinanteils im "Glyzerylcystein" tritt ein gegebenenfalls Aethergruppen aufweisender grösserer Kohlenwasserstoffrest, der maximal nur eine freie oder veresterte Hydroxygruppe, oder höhere alicyclische Kohlenwasserstoffreste aufweisen kann. Die Aminosäurensequenz kann auch verschieden von der oben für die bekannten Degradations-Lipopeptide-Gemische in Formel (II) angegebenen sein, oder an deren Stelle kann auch nur eine einzige Aminosäure vorhanden sein, und es werden Derivate, wie Amide und Ester der terminalen Carboxylgruppe, umfasst. Während in den genannten bekannten Gemischen von Lipopeptiden die Konfiguration am Cystein nur R ist, kann sie bei den Verbindungen gemäss der vorliegenden Anmeldung auch S sein.

Unter den neuen Lipopeptiden gemäss der vorliegenden Erfindung sind besonders solche der Formel (I) hervorzuheben, in denen $R_1$ einen Rest

$$\begin{array}{c} R_3 \\ | \\ CH_2 \\ | \end{array} \qquad (IV)$$

darstellt, worin $R_3$ einen unsubstituierten oder höchstens durch eine freie oder eine mit einer einbasischen Carbonsäure veresterten Hydroxylgruppe substituierten und gegebenenfalls durch eine oder mehrere Sauerstoffatome in der C-Atom-Kette unterbrochenen, gesättigten oder ungesättigten aliphatischen Kohlenwasserstoffrest mit mindestens 12 C-Atomen, der auch durch maximal 2 cycloaliphatische Kohlenwasserstoffreste mit 5 - 8 Ring-C-Atomen substituiert sein kann, bedeutet.

Die genannten aliphatischen Kohlenwasserstoffreste $R_1$ oder $R_3$ bzw. ihre genannten, durch Sauerstoffunktionen substituierten bzw. durch Sauerstoffatome unterbrochenen und/oder mit cycloaliphatischen Kohlenwasserstoffen substituierten Derivate können gesättigt oder ungesättigt, geradkettig oder verzweigt sein, und besitzen insgesamt vorzugsweise nicht mehr als 60 C-Atome. Unter den verzweigten Resten sind insbesondere solche bevorzugt, in denen mindestens eine gerade Kette mit 12-24 C-Atomen vorhanden ist, die gegebenenfalls durch weitere kürzere Ketten substituiert sein kann. In den ungesättigten Resten können mehrere isolierte und/oder konjugierte Doppelbindungen vorhanden sein, vorzugsweise je 1-2 pro gerade Kette, insbesondere 2 konjugierte Doppelbindungen. Vorzugsweise sind die genannten aliphatischen Kohlenwasserstoffreste Alkyl-, Alkenyl- oder Alkadienyl-Reste mit der genannten bevorzugten Konstitution und/oder Anzahl von C-Atomen. Alkylgruppen $R_1$ sind z.B. n-Dodecyl, n-Tridecyl, n-Tetradecyl, n-Pentadecyl, n-Hexadecyl, n-Heneicosyl und n-Docosyl oder die an einem oder mehreren nicht endständigen C-Atomen derselben durch Methyl oder Aethyl substituierten Reste. Alkenylgruppen $R_1$ leiten sich z.B. von diesen Alkylgruppen ab und sind z.B. 11-Dodecenyl, 13-Tetradecenyl, 14-Pentadecenyl, 15-Hexadecenyl, 16-Heptadecenyl, 17-Octadecenyl, 18-Nonadecenyl, 20-Eicosenyl oder einer ihrer isomeren Reste mit der Doppelbindung an anderer Stelle, wie z.B. 9-Octadecenyl, 8-Docosenyl, oder insbesondere das vom Phytol sich ableitende Alkenyl, das 3,7, 11,15-Tetramethyl-2-hexadecenyl. Unter den Resten mit mehreren Doppelbindungen sei z.B. das vom Farnesol sich ableitende Alkatrienyl, das 3,7, 11-Trimethyl-2,6, 10-dodecatrienyl, genannt. Eine Kohlenwasserstoffgruppe $R_1$, wie eine der eben erwähnten, kann auch maximal 2 cycloaliphatische Kohlenwasserstoffreste tragen, die Ringe mit 5-8 C-Atome aufweisen, und unsubstituiert oder durch Niederalkylgruppen mit 1-7 C-Atomen, insbesondere Methylgruppen substituiert sein können. Insbesondere sind solche alicyclischenRinge Cyclohexylringe oder ihre ungesättigten Derivate, wie Cyclohexenyl oder Cyclohexadienyl. Als besonderes Beispiel aus diesen gemischt aliphatisch-cycloaliphatischen Resten sei das 3,7-Dimethyl-9-(2,6,6-trimethyl-1-cyclohexenyl)-2,4,7,8-nona-tetraenyl her-

vorgehoben.

Auch ein Kohlenwasserstoffrest $R_3$ kann eines dieser Alkyl-, Alkenyl-, oder Alka-poly-enyl-Reste sein. Ein Alkylrest $R_3$ ist insbesondere ein solcher der Formel

$$R_4-\underset{|}{\overset{\overset{R_6}{|}}{C}}-R_5 \qquad (V)$$

worin mindestens einer der Substituenten $R_4$, $R_5$, $R_6$ Alkyl, Alkenyl oder Alkadienyl mit einer geraden Kette von 12-24 C-Atomen darstellt, die gegebenenfalls durch Methyl- oder Aethylgruppen substituiert sein können, und die anderen gegebenenfalls Wasserstoff oder Nieder-alkyl- oder Alkenylreste mit 1-7 C-Atomen, wie insbesondere Methyl, Aethyl, Propyl, Butyl, Isopropyl, n-Hexyl oder n-Heptyl bedeuten. Insbesondere können auch hier die genannten langkettigen Alkyl-, Alkenyl- oder Alka-poly-enyl Reste die oben besonders hervorgehobenen sein.

Der Rest $R_1$ kann sodann auch den 5-Cholen-3-yl-Rest darstellen, das heisst den Rest des Cholesterins, der in 3-Stellung an das Schwefelatom des Cysteinanteils in den Verbindungen der Formel (I) gebunden ist. Die genannten Kohlenwasserstoffreste $R_1$ und $R_3$ können auch durch Sauerstoffatome in der C-Atom-Kette unterbrochen sein; sie stellen in diesem Falle Aetherreste dar, wobei eine oder mehrere -O-Gruppen vorhanden sein können, in welch letzterem Falle solche Gruppen durch eine Kette von mindestens 2 C-Atomen getrennt sind. Die bevorzugten Aethergruppen leiten sich ebenfalls von den oben besonders hervorgehobenen Kohlenwasserstoffresten ab. So sind z.B. Polyäthylenglykol-Reste mit der oben für die genannten Alkylgruppen genannten C-Atomzahl in Be-

tracht zu ziehen.

Auch bei den Aethern sind insbesondere Verbindungen der oben genannten Formel (V) besonders bevorzugt, worin sich die Aethergruppen in den genannten langkettigen Alkyl-, Alkenyl-oder Alka-poly-enyl-Reste und/oder in einem der genannten niederaliphatischen Alkyl- oder Alkenylresten mit 1-7 C-Atomen befindet. Bevorzugt sind -O-Gruppen in einem langkettigen Rest $R_4$, $R_5$, $R_6$ dem zentralen angegebenen C-Atom der Formel (V) benachbart und vorzugsweise findet sich maximal je eine -O-Gruppe in einem solchen Rest. Bei den genannten Niederalkyl- oder Alkenyl-Resten sind Aethergruppen bevorzugt am freien Ende der Kohlenstoffkette, insbesondere als Methoxygruppen. Der Rest $R_1$ in Formel (I) kann auch eine Gruppe $-CO-R_1'$ bedeuten, worin $R_1'$ für einen gesättigten oder ungesättigten, aliphatischen oder gemischt aliphatisch-cycloaliphatischen Kohlenwasserstoffrest mit 11-21 C-Atomen steht. Insbesondere kann dieser Rest einer der für Formel (IV) oder (V) genannten Reste sein, wobei jedoch die Zahl der C-Atome höchstens 21 ist. Die bevorzugten Reste sind ebenfalls die oben für die Gruppen der Formeln (IV) und (V) hervorgehobenen. Diese Reste können ebenfalls in der C-Atom-Kette durch -O-Gruppen unterbrochen sein, und die so gebildeten Aethergruppen können wiederum die oben besonders hervorgehobenen sein. Schliesslich kann ein Rest $R_1'$ auch einen der im folgenden beschriebenen Reste gemäss Formel (VI) oder (VIII) darstellen.

Die Erfindung betrifft insbesondere Verbindungen gemäss Formel (I) und ihre Derivate, in welchen der Rest $R_1$ folgende Formel aufweist

$$\begin{array}{l} R_7 \\ | \\ CH - O - X_1 \\ | \\ CH - O - X_2 \\ | \\ CH_2 \\ | \end{array} \qquad \text{(VI)}$$

worin $X_1$ und $X_2$ je Alkyl oder Alkenyl mit 12-20 C-Atomen, $R_7$ Wasserstoff oder Alkyl oder Alkenyl mit 12-20 C-Atomen oder den Rest $-CH_2-O-X_3$ bedeutet, wobei $X_3$ Alkyl oder Alkenyl mit 12-20 C-Atomen bedeuten, wobei als Alkyl oder Alkenyl insbesondere eine der oben hervorgehobenen Gruppen in Betracht fallen.

Insbesondere sind die Verbindungen aus dieser Gruppe von Interesse, in welchen $R_7$ Wasserstoff oder den genannten Rest $-CH_2-O-X_3$ darstellt. Die Alkyl- oder Alkenyl-Gruppen $X_1$, $X_2$, $X_3$ können miteinander identisch oder voneinander verschieden sein. Bevorzugt sind Verbindungen, in denen beide Gruppen $X_1$, $X_2$ bzw. alle drei Gruppen $X_1$, $X_2$, $X_3$ gleich sind, und diese vorzugsweise Hexadecyl, Octadecyl oder Docosanyl sind. In einem oder mehreren der obigen Reste $X_1$, $X_2$, $X_3$ kann auch eine weitere -O-Gruppe vorhanden sein, insbesondere so, dass eine endständige Methoxy- oder Alkoxygruppe in

diesen Resten gebildet wird.

Eine andere bevorzugte Gruppe von Aethern, die den Rest $R_3$ in Formel IV bedeuten können, sind solche der Formel

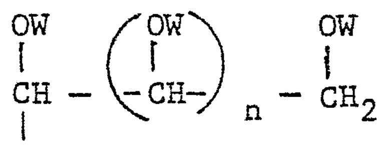

(VII)

worin W je einen niederaliphatischen Kohlenwasserstoffrest mit 1-4 C-Atomen bedeutet und n = 1-3 ist, mit der Massgabe, entsprechend der oben gegebenen Definition von $R_3$, dass die Gesamtzahl der C-Atome mindestens 9 sei. Für den Fall, dass n > 1 ist, sind die Gruppen W insbesondere Methyl.

Der Rest $R_1$ kann auch durch maximal eine freie oder eine mit einer Monocarbonsäure veresterten Hydroxygruppe substituiert sein. Als Monocarbonsäuren kommen insbesondere solche der aliphatischen Reihe in Betracht, insbesondere niederaliphatische Carbonsäuren mit 1-7 C-Atomen, wie z.B. die Essig- oder Propionsäure, die Buttersäuren, Valeriansäuren, Capronsäuren. Ferner kann sich die veresterte Hydroxygruppe von cycloaliphatischen oder cycloaliphatisch-aliphatischen Carbonsäuren, wie z.B. der Cyclopropan-, Cyclobutan-, Cyclopentan- oder Cyclohexancarbonsäure, der Cyclopropyl- oder Cyclobutylmethancarbonsäure oder Cyclopentyl- oder Cyclohexyläthancarbonsäure ableiten.

In erster Linie ist jedoch die genannte veresterte Hydroxygruppe eine sich von einer der unten im Zusammenhang mit der Gruppe $R_2$ angeführten länger-kettigen aliphatischen Carbonsäuren ableitende Gruppe.

Die freie oder veresterte Hydroxylgruppe kann als Substituent auch in Resten $R_1$ oder $R_3$ vorhanden sein, die bereits, wie oben dargestellt, Aethergruppen aufweisen.

Besonders hervorzuheben sind unter den Verbindungen gemäss Formel (I) mit einem der eben genannten Esterreste solche, in denen $R_1$ die Formel

$$\begin{array}{l} X_6 \\ | \\ CH - X_5 \\ | \\ CH - O \,.\, CO \,.\, X_4 \\ | \\ CH_2 \\ | \end{array} \qquad (VIII)$$

besitzt, worin $X_4$ - $X_6$ je Alkyl- oder Alkenyl mit 12-20 C-Atomen, $X_6$ aber auch Wasserstoff sein kann, wobei als Alkyl oder Alkenyl insbesondere eine der oben hervorgehobenen Gruppen in Betracht kommen. Auch entsprechende Verbindungen mit einer freien Hydroxygruppe anstelle der Acyloxy-Gruppe sind von Interesse.

Im Acylrest $R_2$ CO in den Verbindungen gemäss Formel I und ihren Derivaten oder Gemischen ist $R_2$ ein gesättigter oder ungesättigter, aliphatischer oder aliphatischcycloaliphatischer, gegebenenfalls auch durch Sauerstofffunktionen substituierter Kohlenwasserstoffrest mit 11-21 C-Atomen, d.h. der Acylrest leitet sich von gesättigten oder ungesättigten, aliphatischen, oder cycloaliphatisch-aliphatischen, gegebenenfalls im Kohlenwasserstoffrest oxygenierten Carbonsäuren mit 12-22, vorzugsweise mit 14-18 C-Atomen ab. Als solche sind die gesättigten oder ungesättigten Fettsäuren zu nennen, wie Laurinsäure, Myristinsäure, Palmitinsäure, Margarinensäure, Stearinsäure, Arachinsäure, Oelsäure, Elaidinsäure, Linolsäure, α- und β-Eläostearinsäure, Stearolsäure, α-Linolensäure, ferner unter den cycloaliphatisch-aliphatischen z.B. die Dihydrosterkulsäure, Malvalsäure, Hydnocarpussäure und Chaulmoograsäure. Oxygenierte Säuren dieses Typus, welche ebenfalls für den Acylrest in ——————— Betracht kommen, sind z.B. die durch Epoxydierung der

oben genannten olefinischen Fettsäuren und cycloaliphatisch-aliphatischen Säuren sich ergebenden, z.B. die ϑ,ι -Epoxystearinsäure, ferner Derivate der oben genannten Säuren, die z.B. eine oder mehrere Hydroxygruppen aufweisen, wie z.B. die Ricinolsäure.

Eine Aminosäure X oder die Aminosäuren einer Sequenz X gemäss Formel (I) sind beliebige D- oder L-Aminosäuren, in denen Aminogruppen und Carboxylgruppen an aliphatische C-Atome gebunden sind, d.h. eines, das nicht Teil eines aromatischen Systems ist. Insbesondere kommen "natürliche" Aminosäuren, d.h. in der Natur vorkommende, ihre Antipoden und Analogen in Betracht. DiePeptidsequenz X in den Verbindungen der Formel (I) bzw. in deren Salzen setzt sich aus maximal 10 Aminosäuren zusammen, wobei vorzugsweise mindestens die Hälfte eine hydrophile Gruppe tragen, wie insbesondere Hydroxy-, Amino-, Carboxyl-, Carbamid-, Guanidino- oder Imidazolylgruppen. Von den ionischen Aminosäuren dieser Kategorie seien unter den saure Gruppen tragenden, insbesondere die Asparagin- und Glutamin- und die Oxyglutaminsäure hervorgehoben, unter den basische Gruppen tragenden das Lysin, das Ornithin, das Arginin und das Histidin. Aminosäuren mit neutralem Charakter sind besonders die Amide, wie das Asparagin, das Glutamin und die Hydroxygruppen tragenden, in erster Linie Serin und Threonin.

Stellt X in Formel (I) die genannte Aminosäure dar, so ist sie ebenfalls z.B. eine der genannten hydrophile Gruppen tragenden, z.B. insbesondere Serin oder Threonin.

Unter den Aminosäuren, die in die obige Sequenz eingehen können und die keine hydrophilen Gruppen tragen,

sind vor allem die unsubstituierten zu erwähnen, wie z.B. Glycin, Alanin, Valin, Norvalin, Leucin, Isoleucin, Phenylalanin, sodann aber auch einige substituierte, die nicht-hydrophilen Charakter haben, wie Methionin.

Die Reihenfolge der genannten Aminosäuren in der Peptidkette X kann beliebig sein, jedoch sind solche Sequenzen bevorzugt, in denen sämtliche Aminosäuren mit hydrophilen Gruppen direkt aneinander gebunden sind, und unter diesen wiederum diejenigen, in welchen diese Sequenz der hydrophilen Aminosäuren an die Carboxylgruppe des substituierten Cystein- bzw. Homo-Cystein-Teils gebunden ist.

Eine wichtige Gruppe von Lipopeptiden gemäss der vorliegenden Erfindung umfasst Verbindungen, in denen die Peptidkette X aus maximal 5 Aminosäuren besteht. Unter diesen sind insbesondere solche der Formel

* = S oder R
oder S/R

** = S oder R
oder S/R

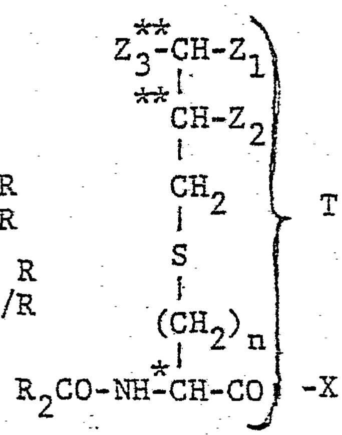

T

IX

n = 1,2

worin $Z_1$, $Z_2$, $Z_3$ Alkyl oder Alkenyl mit 12-20 C-Atomen, oder $Z_1$ und $Z_2$ die Reste $-O-Z_1'$, bzw. $-O-Z_2'$ bedeuten, wo $Z_1'$ und $Z_2'$ ebenfalls Alkyl oder Alkenyl mit 12-20 C-Atomen darstellen, und $Z_3$ = H oder einen Rest $-CH_2-O-Z_3'$, worin $Z_3'$ Alkyl oder Alkenyl mit 12-20 C-Atomen bedeutet, und X die für Formel (I) gegebene Bedeutung hat, aber im Falle der Aminosäurensequenz 2-5 Aminosäuren aufweist, in welcher vorzugsweise mindestens die Hälfte eine hydrophile Gruppe tragen, und dies auch

für den Fall einer einzigen Aminosäure zutrifft, sowie ihre Salze und Komplexe. Unter diesen Lipopeptiden seien insbesondere folgende Lipopeptid-Typen erwähnt:

(T gemäss Formel IX)

T - Ser - Ser - Asn - Ala - Lys - OH
T - Ser - Ser - Asn - Ala - OH
T - Ser - Ser - Asn - OH
T - Ser - Ser - OH,

sodann die entsprechenden Typen, in welchen Threonin, Glutamin oder Asparagin das Serin ersetzen; ferner die Verbindungstypen der Art:

T - Ser - Ser - Asn - Ala - Glu - OH
T - Ser - Ser - Phe - Ala - Glu - OH
T - Ser - Ser - Phe - Ala - OH
T - Ser - Ser - Phe - OH

und die entsprechenden mit Threonin, Glutamin oder Asparagin als Austauschaminosäuren für Serin, ferner

T - Phe - Phe - Asn - Ala - Lys - OH
T - Glu - Gln - Asn - Ala - Lys - OH
T - Ser - Ser - Asn - Ala - Glu - OH
T - Ser - Ser - Asn - Ala - Ala - OH
T - Val - Lys - Val - Tyr - Pro - OH
T - Phe - Ile - Ile - Phe - Ala - OH
T - Ser - Ser - Phe - Ala - Glu - OH
T - Ser - Thr - Phe - Ala - Glu - OH
T - Ser - Phe - Ala - Glu - OH
T - Thr - Thr - Phe - Ala - Glu - OH
T - Ser - Ser - Asn - Ala - Lys - OH
T - Ser - Ser - Asn - Ala - D-Glu - OH

T - Ser - Ser - D-Asn - Ala - Glu - OH
T - Thr - Thr - Asn - Ala - Lys - OH
T - Ser - Thr - Asn - D-Ala - Lys - OH
T - Ser - Ser - Asn - Ala - Glu - OH
T-D-Ser - Ser - Phe - Ala - D-Glu - OH

und die Amide und Carbonsäureester mit terminaler Carbamid- bzw. Estergruppe, wobei in erster Linie die Verbindungen in Betracht zu ziehen sind, in denen der Acylrest $R_2$-CO- Palmitoyl, Stearoyl oder Oleoyl, $Z_3$ Wasserstoff und $Z_1$ und $Z_2$ Alkoxyreste darstellen, und dieselben untereinander gleich sind und vorzugsweise Hexadecyloxy, oder Octadecyloxy darstellen, und diejenigen, in denen $Z_1$ und $Z_2$ verschieden sind und die genannten Alkoxyreste bedeuten, und diese Reste in beliebiger Kombination und/oder Variation vorkommen. Es seien als Beispiele genannt:

Verbindungen gemäss Formel IX, worin

$R_2CO$ = Palmitoyl, $Z_1$ und $Z_2$ = Hexadecyloxy $Z_3$=H, wobei X eine der folgenden Sequenzen sein kann:

Ser - Ser - Asn - Ala - Lys - OH
Ser - Ser - Asn - Ala - OH
Ser - Ser - Asn - OH
Ser - Ser - OH
Ser - OH, oder Verbindungen gemäss Formel IX, worin $R_2CO$ = Stearoyl, $Z_1$ und $Z_2$ = Octadecyloxy $Z_3$=H, oder $R_2CO$ = Dihydrosterkuloyl, $Z_1$ und $Z_2$ = Hexadecyloxy, $Z_3$=H, oder $R_2CO$ = Lauroyl, $Z_1$ und $Z_2$ = octadecenyloxy, $Z_3$=H, oder $R_2CO$ = Lauroyl, $Z_1$ und $Z_2$ = Hexadecyloxy, $Z_3$=H, bei welchen X eine beliebige der oben für das N-Palmitoyl-Derivat beschriebenen Sequenzen sein kann, ferner die Verbindungen, die sich aus der Substitution des Serins durch Threonin, Glutaminsäure, Glutamin, Asparaginsäure oder Asparagin und aus der Substitution des Lysins durch

Glutaminsäure und Asparagin durch Phenylalanin ergeben.

All die genannten Verbindungen gemäss Formel IX können sowohl am * C, wie an jedem der ** C die absolute R oder S oder S/R-Konfiguration aufweisen. Insbesondere umfasst die Erfindung auch die Gemische von Diastereomeren, die sich aus der Variation dieser möglichen Konfigurationen ergeben. Besonders zu erwähnen ist als Untergruppe diejenige mit der R-Konfiguration an den ** C-Atomen. Eine zweite Gruppe von Lipopeptiden gemäss der vorliegenden Erfindung umfasst Verbindungen gemäss der Formel IX, in denen jedoch X eine Aminosäurensequenz mit 6-10 Aminosäuren darstellt, wobei die ersten 5 vorzugsweise die oben bereits hervorgehobenen Sequenzen darstellen. Die Aminosäuren 6-10 können wiederum beliebige der oben erwähnten sein, wie insbesondere Serin, Asparagin, Alanin, Glutaminsäure, Lysin, Phenylalanin und in etwa folgender Sequenz vorkommen:

- Ser - Ser - Asn - Ala - Lys - Ile - Asp - Glu - OH
- Ser - Ser - Asn - Ala - Lys - Asp - Glu - OH

Spezifische Verbindungen gemäss Formel IX mit mehr als 5-Aminosäuren in der Sequenz X sind z.B. solche, in denen $R_2$-CO = Dihydrosterkuloyl, $Z_1$ und $Z_2$ = Hexadecyloxy, $Z_3$=H, und X = Ser - Ser - Asn - Ala - Glu - Ile - Asp-Glu - OH, oder

$R_2$-CO = Stearoyl, $Z_1$ und $Z_2$ = Hexadecyloxy, $Z_3$=H, und X = Ser - Ser - Asn - Ala - Glu - Ile - Asp - Glu - OH oder

$R_2$-CO = Palmitoyl, $Z_1$ und $Z_2$ = Hexadecyloxy, $Z_3$=H, und X = Ser - Ser - Asn - Ala - Glu - Ile - Asp - Glu - OH, oder $R_2$-CO = Palmitoyl, $Z_1$ und $Z_2$ = Octadecyloxy, $Z_3$=H, und X = Ser - Ser - Asn - Ala - Lys - Ile - Asp - Glu - OH.

Die für Formel IX angeführten spezifischen Bedeutungen für die Aminosäure X oder die Sequenzen X sind auch für

die Verbindungen gemäss der Formel (I) die bevorzugten und auch für die Verbindungen dieser Formel, in welcher $R_1$ eine der Partialformeln IV - VIII besitzt.

In den oben besprochenen neuen Lipopeptiden gemäss der vorliegenden Erfindung kann die terminale Carboxylgruppe in amidierter Form vorliegen. Ausser dem unsubstituierten Amid kommen auch solche in Betracht, die sich von einem primären oder sekundären Amin ableiten. Insbesondere kommen Amide von niederen aliphatischen Aminen mit 1-7 C-Atomen in Betracht, wie Methylamin, Ethylamin, Propylamin, Butylamin, Dimethylamin, Diäthylamin oder Propyl- und Isopropylamin, ferner aromatische Amine, insbesondere monocyclische, wie Anilin oder Toluidin, araliphatische, wie Benzylamin oder heterocyclische Amine, wie z.B. die Aminopyridine. Bei den sekundären aliphatischen Aminen kann es sich auch um ringgeschlossene Stickstoffbasen handeln, wie z.B. Pyrrolidin, Piperidin oder Piperazin. Spezifische Amide sind z.B. die unsubstituierten oder die Methyl-, Aethyl-, Dimethyl- oder Diäthylamide sämtlicher oben angeführter spezifischer Lipopeptide gemäss der vorliegenden Erfindung oder derartige Amide der in den illustrativen Beispielen beschriebenen Verbindungen.

In den veresterten terminalen Carboxylgruppen der neuen Lipopeptide leitet sich die Alkoholkomponente vorzugsweise von niederen aliphatischen Alkoholen mit 1-7 C-Atomen, wie Methyl-, Ethyl-, n-Propyl-, Isopropyl- oder den Butylalkoholen ab. Die veresternden Alkohole können aber auch mehrwertige sein, wie Aethylenglykol oder Propylenglykol oder Glyzerin. Auch araliphatische Alkohole, insbesondere monocyclische niederaliphatische mit 1-7 C-Atomen im aliphatischen Teil, wie z.B. Benzylalkohol, oder heterocyclische Alkohole, wie Tetrahydrofuranol oder Tetrahydropyranol können für die Veresterung verwendet werden. Als spezifische Ester dieses Typs der Lipopeptide gemäss der Erfindung seien z.B. die Methyl-, Aethyl- und die Aethylenglykol- oder Propylenglykol-Ester sämtlicher oben angeführter spezifischer Lipopeptide oder derjenigen, die in den illustrativen Beispielen beschrieben sind, erwähnt.

Die vorliegenden neuen Lipopeptide sind je nach der Art ihrer Substituenten neutrale, saure oder basische Verbindungen. Falls überschüssige saure Gruppen vorhanden sind, bilden sie Salze mit Basen, wie Ammoniumsalze oder Salze mit Alkali- oder Erdalkalimetallen, z.B. Natrium, Kalium, Calcium oder Magnesium; sind jedoch überschüssige basische Gruppen vorhanden, bilden sie Säureadditionssalze.

Säureadditionssalze sind besonders pharmazeutisch verwendbare, nichttoxische Säureadditionssalze, wie solche mit anorganischen Säuren, z.B. Chlorwasserstoff-, Bromwasserstoff-, Salpeter-, Schwefel- oder Phosphorsäuren, oder mit organischen Säuren, wie organischen Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organischen Sulfonsäuren, z.B. Methansulfonsäure, Aethansulfonsäure, 2-Hydroxy-äthansulfonsäure, Aethan-1,2-disulfonsäure, Benzolsulfonsäure, p-Toluolsulfonsäure oder Naphthalin-2-sulfonsäure, ferner auch andere Säureadditionssalze, die z.B. als Zwischenprodukte, z.B. zur Reinigung der freien Verbindungen oder in der Herstellung von anderen Salzen, sowie zur Charakterisierung verwendet werden können, wie z.B. solche mit Pikrin-, Pikrolon-, Flavian-, Phosphorwolfram-, Phosphormolyddän-, Chlorplatin-, Reinecke- oder Perchlorsäure.

Komplexe sind die mit Metallsalzen, z.B. mit Schwermetallsalzen, wie Kupfer, Zink, Eisen oder Kobaltsalzen entstehenden Verbindungen. Zur Bildung solcher Komplexe werden vorzugsweise die Phosphate, Pyrophosphate und Polyphosphate dieser Metallsalze verwendet, gegebenenfalls in Kombination mit sauren organischen Stoffen, z.B. saure Gruppen enthaltenden Polysacchariden, wie Carboxymethylcellulose, Gerbsäure, Polyglutaminsäure oder teilweise hydrolysierte Gelatine, ferner Alkalimetallpolyphosphate wie z.B. "Calgon N", "Calgon 322", "Calgon 188" oder "Plyron B 12".

Die Verbindungen der vorliegenden Erfindung gemäss der obigen Formel (I), ihre Salze und Komplexe und Gemische weisen wertvolle pharmakologische Eigenschaften, insbesondere eine ausgeprägte immunpotenzierende Wirkung auf. So stimulieren die Verbindungen im Dosisbereich von 0,5-320 µg/ml die in vitro an Hand der Thymidininkorporation bestimmte Proliferation von B-Lymphocyten 20- bis 50-fach im Vergleich zu nicht stimulierten Kontroll-Lymphocyten. Das Ausmass der Stimulation entspricht demjenigen, das mit den wirksamsten bekannten B-Zell-Mitogenen [ Dextran-sulfat, E. coli Lipopolysaccharid, PPD (purified protein derivative)] erreicht wird, wobei bei den neuen Verbindungen der vorliegenden Anmeldung auch hohe Konzentrationen nicht lymphocytotoxisch wirken.

Die neuen Verbindungen der Formel (I), ihre Salze und Komplexe und Gemische sind ausserdem in der Lage, in Konzentrationen von 0,3-60 µg/ml in Milzzellkulturen von normalen Mäusen die Bildung antikörperproduzierender Zellen zu induzieren (Vermehrung der 19S-plaquebildenden

Zellen um einen Faktor 20 bis 50 über den Kontrollwert (in Abwesenheit der stimulierenden Substanzen)): So werden in Anwesenheit der genannten Verbindungen z.B. spezifische Antikörper gegen Schaferythrocyten gebildet, ohne dass den Kulturen Schaferythrocyten zur Immunisierung zugesetzt werden. Andererseits vermögen die genannten Substanzen im selben Konzentrationsbereich auch die immunologische Reaktivität von T-zellverarmten Milzzellkulturen (von kongenital athymischen nu/nu Mäusen) gegenüber einem normalerweise thymusabhängigen Antigen (Schaferythrocyten) zu steigern (Faktor 10 bis 40 gegenüber unbehandelten Kontrollkulturen). Durch die genannten Verbindungen werden aber in vitro direkt oder indirekt nicht nur Proliferations- und Syntheseleistungen von B-Lymphocyten (d. h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören), vermittelt. So vermögen z.B. die erwähnten Verbindungen in einem Konzentrationsbereich von 10-100 µg/ml die Reaktivität von cortisonresistenten Thymuszellen gegenüber allogenen bestrahlten Stimulatorlymphocyten erheblich (bis zu 10-fach) zu potenzieren.

Die oben erwähnten Wirkungen kommen wahrscheinlich indirekt dadurch zustande, dass die Lipopeptide Makrophagen aktivieren, die ihrerseits die Reaktivität von T- und B-Lymphocyten fördern. Tatsächlich kann man zeigen, dass die genannten Verbindungen bereits in äusserst geringen Konzentrationen (0,01 - 10 µg/ml) grosse Mengen "colony stimulating activity" (CSA) aus Maus-Makrophagen freisetzen (Induktion von bis zu 150 - 200 Kolonien innert 7 Tagen aus $10^5$ Mäuse-Knochenmarkzellen, nach Zugabe von 20% Ueberstand aus während 24 Stunden mit Substanz

inkubierten Makrophagenkulturen, im Vergleich zu 0 - 5 Kolonien bei Zugabe von Ueberständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die genannten Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirkung der neuen Verbindungen der Formel (I), ihrer Salze oder Komplexe oder Gemische, kann auch in vivo nachgewiesen werden: So führt die Injektion eines Lipopeptids gemäss der Erfindung innert 3-9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5% Endkonzentration] im Vergleich zu 0 - 5 Kolonien bei unbehandelten Tieren). Dementsprechend wird durch Verabreichung derselben Verbindungen in vivo die Antikörperbildungsfähigkeit von Mäusen erheblich potenziert:

NMRI Mäuse werden durch intraperitoneale Injektion von 10 µg präzipitatfreiem BSA am Tag 0 immunisiert. 9,15 und 29 Tage später werden Serumproben entnommen und auf ihren Gehalt an anti-BSA-Antikörpern mit einer passiven Haemagglutinationstechnik untersucht. In der verwendeten Dosis ist lösliches BSA für die Empfängertiere subimmunogen, d.h. es vermag keine oder nur eine ganz geringfügige Produktion von Antikörpern auszulösen. Zusätzliche Behandlung der Mäuse mit gewissen immunpotenzierenden Stoffen vor oder nach der Antigengabe führt zu einem Anstieg der Antikörpertiter im Serum. Der Effekt der Behandlung wird durch den erreichten Scorewert, d.h.

durch die Summe der $log_2$ Titerdifferenzen an den drei Blutungstagen ausgedrückt.
In diesem Test sind die Verbindungen der Formel (I), ihre Salze oder Komplexe oder Gemische in der Lage bei intraperitonealer oder subkutaner Applikation von 0,03-3 mg/kg Tier an fünf aufeinander folgenden Tagen vor oder nach Immunisierung mit BSA die Antikörperproduktion gegen BSA signifikant zu steigern.

Auch Manifestationen der zellvermittelten Immunität können durch die genannten Verbindungen in vivo potenziert werden:

Während Sensibilisierung von Meerschweinchen mit BSA in inkomplettem Freund'schen Adjuvans nur zu humoraler Antikörperbildung führt, induziert die Beimischung der Lipopeptide gemäss der vorliegenden Erfindung in einem Dosisbereich von 1-15µg zur wässerigen Phase der Antigen-Oelemulsion Spättyp-Ueberempfindlichkeit gegenüber BSA: 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund'schem Adjuvans (d.h. mit Zusatz von Mykobakterien) erhalten werden. Die $ED_{50}$-Werte (benötigte µg/Tier zur Induktion einer Differenz des Reaktionsvolumens (Erythemfläche x Hautdickenzunahme) bei behandelten und unbehandelten Tieren von 200 µl, 24 Stunden nach Auslösung) betragen 2-3 µg.

Die Lipopeptide gemäss der vorliegenden Erfindung sind zudem wenig toxisch: Auch 5-malige intraperitoneale Applikation in einer Dosis von 10 mg/kg/Tag an fünf

aufeinanderfolgenden Tagen wurde von Mäuse anscheinend symptomlos vertragen. Da die für die Immunstimulation benötigten Dosen sehr gering sind, ist die therapeutische Breite der neuen Verbindung sehr gross.

Die neuen Lipopeptide gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Lipopeptide gemäss der vorliegenden Erfindung können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedenen Antigenen als Adjuvantien bei der experimentellen und industriellen Herstellung von Antiseren für Therapie und Diagnostik und bei der Induktion von immunologisch aktivierten Lymphocyten-populationen für Zelltransferverfahren.

Darüberhinaus können die neuen Lipopeptide auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie

mit ionisierenden Strahlen oder mit immunosuppressiv wirkenden Hormonen auftreten. Die genannten Stoffe können somit vorzugsweise auch in Kombination mit Antibiotika, Chemotherapeutika oder anderen Heilverfahren verabreicht werden, um immunologischen Schädigungen entgegenzuwirken. Schliesslich sind die beschriebenen Stoffe auch zur allgemeinen Prophylaxe von Infektionskrankheiten bei Mensch und Tier geeignet.

Die neuen Lipopeptide können nach an sich bekannten Methoden oder deren hier beschriebenen neuen Verfahren hergestellt werden.

Nach einem bevorzugten Verfahren werden die Verbindungen der Formel (I), ihre diastereomeren Gemische und ihre Salze und Komplexe dadurch hergestellt,

a) dass man in einer Verbindung der Formel

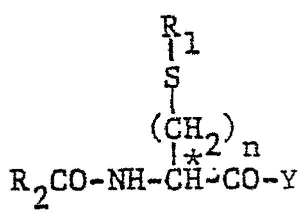

(X)

n = 1,2

* = S/R oder S oder R

worin $R_1$, und $R_2$ die für Formel (I) angegebene Bedeutung haben und Y eine X in der Formel (I) entsprechende Aminosäure oder Aminosäurensequenz darstellt, worin jedoch mindestens eine der die Aminosäuren substituierenden hydrophilen Gruppen und/oder die terminale Carboxylgruppe durch eine unter neutralen oder milden sauren Bedingungen abspaltbare Schutzgruppe geschützt ist, oder in einem Salz einer solchen Verbindung, die Schutzgruppe(n) abspaltet, oder

b) dass man eine Verbindung der Formel

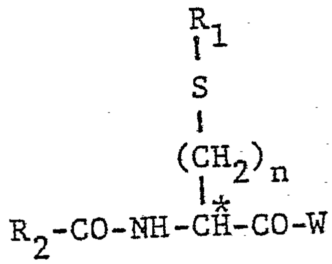

(XI)

n = 1,2

* = S/R oder S oder R

worin $R_1$, und $R_2$ die für Formel (I) angegebene Bedeutung haben und W = OH oder eine Aminosäure oder eine unvollständige Sequenz von Aminosäuren gemäss X in Formel (I) darstellt, mit einer Verbindung der Formel

$$NH_2 - Z_1 \qquad (XII)$$

wo $Z_1$ eine der Gruppe X in Formel (I) entsprechende Gruppe bzw. eine zur genannten Aminosäure oder unvollständigen Sequenz von Aminosäuren gemäss X komplementären Aminosäuresequenz bzw. Aminosäure bedeutet, in welchen Sequenzen jedoch keine freien Aminogruppen vorhanden sind, oder einem Salz derselben, umsetzt, oder

c) in einer Verbindung entsprechend derjenigen der Formel I, in welcher im Rest $R_1$ mindestens eine freie Hydroxygruppe vorhanden ist, und im Rest X aber keine freien Hydroxygruppen vorhanden sind, die freien Hydroxygruppen veräthert, oder

d) in einer Verbindung gemäss Formel (I), worin der Rest $R_1$ eine freie Hydroxygruppe aufweist, aber keine freien Hydroxylgruppen in X vorhanden sind, die freie Hydroxygruppe mittels eines acylierenden Mittels verestert, oder

e) eine Verbindung der Formel

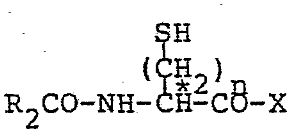

n = 1,2

* = S/R oder S oder R

(XIII) ,

worin $R_2$ und X die oben angegebene Bedeutung haben, aber keine freien Hydroxygruppen in X vorhanden sind, mit einem acylierenden Mittel behandelt, oder

f) eine Verbindung der Formel

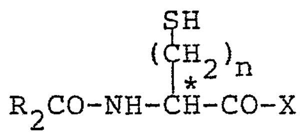

n = 1,2 (XIV),

* = S/R oder S oder R

worin $R_2$ und X die gleiche Bedeutung wie in Formel I haben, mit einer Verbindung der Formel

$$R_1 - T \qquad (XV),$$

worin T eine reaktionsfähige funktionell abgewandelte Hydroxygruppe bedeutet, in Gegenwart eines basischen Mittels umsetzt, oder

g) eine Verbindung der Formel

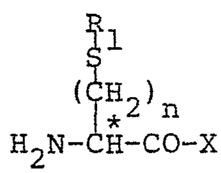

n = 1,2

* = S/R oder S oder R (XVI),

worin X und $R_1$ die gleiche Bedeutung wie in Formel I haben, jedoch keine Hydroxylgruppen und Aminogruppen in X vorhanden sind, mit einem Acylierungsmittel behandelt,

und, wenn erwünscht, in erhaltenen Verbindungen mit freier terminaler Carboxylgruppe dieselbe in eine Amidgruppe oder Estergruppe überführt, und/oder die Verbindungen in ihre Salze oder Komplexe überführt.

Die Schutzgruppen im Verfahren gemäss Variante a) sind besonders die von der Synthese von Peptiden her bekannten.

So sind z.B. Schutzgruppen für Aminogruppen Acyl- oder Aralkylgruppen wie Formyl-, Trifluoracetyl-, Phthaloyl-, Benzolsulfonyl-, p-Toluolsulfonyl-, o-Nitrophenylsulfenyl-, 2,4-Dinitrophenylsulfenylgruppen (diese Sulfenylgruppen können auch durch Einwirkung nucleophiler Reagenzien, z.B. Sulfite, Thiosulfate, abgespalten werden), gegebenenfalls substituierte, wie z.B. durch Niederalkoxygruppen, besonders o- oder p-Methoxygruppen substituierte Benzyl-, oder Diphenyl- oder Triphenylmethylgruppen oder von der Kohlensäure sich ableitende Gruppen, wie gegebenenfalls in den aromatischen Ringen, z.B. durch Halogenatome wie Chlor oder Brom, Nitrogruppen, Niederalkyl- oder Niederalkoxygruppen oder farbgebende Gruppen, z.B. Azogruppen substituierte Arylmethyloxycarbonylgruppen, in denen die Methylengruppe durch einen weiteren Arylrest und/oder einen oder gegebenenfalls zwei niedere Alkylreste substituiert sein kann, wie Benzyl-, Benzhydryl- oder 2-Phenyl-isopropyloxycarbonylgruppen, z.B. Carbobenzoxy, p-Brom- oder p-Chlorcarbobenzoxy, p-Nitrocarbobenzoxy oder p-Methoxycarbobenzoxy, p-Phenylazo-benzyloxycarbonyl und p-(p'-Methoxy-phenylazo)-benzyloxycarbonyl, 2-Tolyl-isopropyloxycarbonyl und insbesondere 2-(p-Biphenylyl)-isopropyloxycarbonyl, sowie aliphatische Oxycarbonylgruppen wie Adamantyloxycarbonyl, Cyclopentyloxycarbonyl, Trichloräthyloxycarbonyl, tert. Amyloxycarbonyl oder vor allem tert.-Butyloxycarbonyl.

Die Aminogruppen können auch durch Bildung von Enaminen, erhalten durch Reaktion der Aminogruppe mit 1,3-Diketonen, z.B. Benzoylaceton, Acetylaceton oder Dimedon, geschützt werden.

Carboxylgruppen sind beispielsweise durch Amid- oder Hydrazidbildung oder durch Veresterung geschützt. Die Amid- und Hydrazidgruppen können gegebenenfalls substituiert sein, die Amidgruppe z.B. durch die 3,4-Dimethoxybenzyl- oder bis-(p-Methoxyphenyl)-methylgruppe, die Hydrazidgruppe z.B. durch die Carbobenzoxygruppe, die Trichloräthyloxycarbonylgruppe, die Trifluoracetylgruppe, die Tritylgruppe, die tert.-Butyloxycarbonylgruppe oder die 2-(p-Biphenylyl)-isopropyloxycarbonylgruppe. Zur Veresterung geeignet sind z.B. niedere gegebenenfalls substituierte Alkanole wie Methanol, Aethanol, Cyanmethylalkohol, Benzoylmethylalkohol oder insbesondere tert.-Butanol, ferner Aralkanole wie Arylniederalkanole, z.B. gegebenenfalls durch Niederalkyl- oder Niederalkoxygruppen oder Halogenatome substituierte Benzylalkohole oder Benzhydrole wie Benzhydrol, p-Nitrobenzylalkohol, p-Methoxybenzylalkohol, 2,4,6-Trimethylbenzylalkohol, gegebenenfalls durch elektronenanziehende Substituenten substituierte Phenole und Thiophenole wie Thiophenol, Thiokresol, p-Nitrothiophenol, 2,4,5- und 2,4,6-Trichlorphenol, Pentachlorphenol, p-Nitrophenol, 2,4-Dinitrophenol, p-Cyanophenol oder p-Methansulfonylphenol, weiter z.B. N-Hydroxysuccinimid, N-Hydroxyphthalimid, N-Hydroxypiperidin, 8-Hydroxychinolin.

Die Hydroxygruppen der Serin- und Threoninreste können z.B. durch Veresterung oder Verätherung geschützt sein.

Als Acylreste bei der Veresterung sind vor allem von der Kohlensäure sich ableitende Reste wie Benzoyloxycarbonyl oder Aethyloxycarbonyl geeignet. Zur Verätherung geeignete Gruppen sind z.B. Benzyl-, Tetrahydropyranyl- oder tert.-Butylreste. Ferner eignen sich zum Schutz der Hydroxylgruppen die in Ber. 100 (1967), 3838 - 3849 beschriebenen 2,2,2-Trifluor-1-tert.-butyloxycarbonyl-

amino- oder -1-benzyloxycarbonylaminoäthylgruppen (Weygand).

Vorzugsweise verwendet man zum Schutz der Carboxylgruppe der Seitenketten und gegebenenfalls der terminalen Carboxylgruppe die tert.-Butylestergruppe oder die Benzhydrolgruppe, zum Schutz der Aminogruppen der Seitenketten die tert.-Butyloxycarbonylgruppe, für die Hydroxylgruppen von Serin oder Threonin, die tert.-Butyläthergruppe, und, wenn erwünscht, zum Schutz der Iminogruppe des Histidins die 2,2,2-Trifluor-1-tert.-butyloxycarbonylamino-äthylgruppe.

Die verfahrensgemässe Abspaltung der Schutzgruppen mit sauren Mitteln unter milden Bedingungen erfolgt z.B. in einer von der Peptidchemie her bekannten Weise, z.B. durch Behandlung mit Trifluoressigsäure.

Eine besondere Schutzgruppe für Carboxylgruppen, welche unter neutralen Bedingungen abspaltbar ist, ist die z.B. in der Deutschen Offenlegungsschrift 27 06 490 beschriebene Gruppe der allgemeinen Formel

$$R^2 - \overset{\displaystyle R_1}{\underset{\displaystyle R^3}{Si}} - CH_2 - CH_2 -$$

worin $R^1$, $R^2$ und $R^3$ je einen Kohlenwasserstoffrest bedeutet, wobei die Reste untereinander auch durch eine einfache C-C Bindung verknüpft sein können, insbesondere Alkylreste mit 1-5 C Atomen. Schutzgruppen dieses Typs sind z.B. die 2-(Dimethyl-tert.-butylsilyl)-äthyl-, die 2-(Dibutyl-methyl-silyl)-äthyl- und vor allem die 2-(Trimethylsilyl)-äthylgruppe. Obwohl diese Schutzgruppen auch basisch abgespalten werden können, ist besonders ihre Abspaltbarkeit unter neutralen Bedingungen, und

zwar durch Einwirkung eines Salzes der Fluorwasserstoffsäure, von Interesse. Die Abspaltung der Schutzgruppe wird vorteilhaft in einem aprotischen organischen Lösungsmittel vorgenommen; vorzugsweise wird dabei die Anwesenheit von solchen Lösungsmitteln vermieden, welche das Fluorid-Anion zu solvatisieren vermögen, wie Wasser oder niederaliphatische Alkohole.

Die Kondensation gemäss der Variante b) der Verbindung der Formel (XI) mit der Verbindung der Formel (XII) erfolgt z.B. in der Weise, dass man die Verbindung (XI) in Form der aktivierten Carbonsäure mit (XII) umsetzt, oder dass man die Säure (XI) mit der Verbindung (XII), deren Aminogruppe in aktivierter Form vorliegt, umsetzt. Gegebenenfalls werden die Verbindungen (XI) bzw. (XII) für die Kondensation in Form ihrer Salze eingesetzt.

Die Carboxylgruppe der Verbindung (XI) kann beispielsweise durch Ueberführung in ein Säureazid, -anhydrid, -imidazolid, -isoxazolid oder einen aktivierten Ester, wie Cyanmethylester, Carboxymethylester, Thiophenylester, p-Nitrothiophenylester, Thiokresylester, p-Methansulfonylphenylester, p-Nitrophenylester, 2,4-Dinitrophenylester, 2,4,5- oder 2,4,6-Trichlorphenylester, Pentachlorphenylester, N-Hydroxysuccinimidester, N-Hydroxyphthalimidester, 8-Hydroxychinolinester, 2-Hydroxy-1,2-dihydro-1-carbäthoxy-chinolin-ester, N-Hydroxypiperidinester oder einen Enolester, der mit N-Aethyl-5-phenyl-isoxazolium-3-sulfonat gewonnen wird [Woodward Reagens], oder durch Reaktion mittels eines Carbodiimids (gegebenenfalls unter Zusatz von N-Hydroxysuccinimid) oder eines unsubstituierten oder z.B. durch Halogen, Methyl oder Methoxy substituierten 1-Hydroxybenzotriazols, 3-Hydroxy-4-oxo-3,4-dihydrobenzo[d]-1,2,3-triazins oder N,N'-Carbonyldiimidazols, aktiviert werden.

Die Aminogruppe der Verbindung (XII) kann bei-

spielsweise durch Reaktion mit einem Phosphit aktiviert werden.

Als gebräuchlichste Methoden der Kondensation sind zu nennen: die Methode nach Weygand-Wünsch (Carbodiimid in Gegenwart von N-Hydroxysuccinimid), die Azidmethode, die Methode der N-Carboxyanhydride oder N-Thiocarboxyanhydride, die Methode der aktivierten Ester und die Anhydridmethode. Insbesondere können diese Kondensationen nach der Merrifield-Methode vollzogen werden.

Es ist auch möglich, die Kondensation der Verbindung (XI) nach den eben erwähnten Methoden mit einer Verbindung entsprechend der Formel (XII) zu vollziehen, in welcher die terminale Carboxylgruppe durch eine von einer, wie für X definiert, Amid- oder Estergruppe verschiedene Schutzgruppe, wie eine der oben angegebenen, blockiert ist, und/oder eine oder mehrere der vorhandenen hydrophilen Gruppen in geschützter Form, wie oben beschrieben, vorliegen. Man gelangt so zu Kondensationsprodukten, welche zur Gruppe der für die oben beschriebene Variante a) benützten Ausgangsstoffe der Formel (X) gehören, die ebenfalls Gegenstand der vorliegenden Erfindung sind.

Gemäss den obigen Varianten c), d) und e) werden in Verbindungen der Formel (I), in denen jedoch in X keine freien Hydroxygruppen vorhanden sind, freie Hydroxygruppen in $R_1$ bzw. die SH-Gruppe in Verbindungen der Formel XIII veräthert bzw. acyliert. Die Acylierung kann in an sich bekannter Weise ausgeführt werden, z.B. durch Umsetzung mit einem reaktionsfähigen funktionellen Derivat der dem einzuführenden Rest entsprechenden Carbonsäure, wie z.B. einem Anhydrid oder Säurehalogenid, vorzugsweise in Gegenwart einer tertiären Base, wie Pyridin oder Kollidin. Freie Aminogruppen im Teil würden dabei auch verestert, so dass dieselben vor der Acylierung gegebenenfalls geschützt werden müssen, z.B. durch Ueberführung in ein Salz, z.B. in das Hydrochlorid.

Dieses Verfahren eignet sich besonders zur Darstellung von Verbindungen gemäss Formel (I), worin die Acylgruppen im Rest $R_1$ verschieden vom Acylrest $R_2$ sind.

Die Verätherung freier Hydroxygruppen kann ebenfalls in an sich bekannter Weise ausgeführt werden, z.B. durch Behandlung mit einem Alkyl- oder Alkenylhalogenid in Gegenwart einer Base, z.B. von Silberoxid oder, insbesondere mit einem Diazoalkan, wie einem niederaliphatischen Diazoalkan mit 1-3 C-Atomen, in erster Linie Diazomethan, z.B. in Aetherlösung. Diese Methode eignet sich insbesondere zur Herstellung von Verbindungen mit einem Rest $R_1$ gemäss der Partialformel (VII).

Werden diese Aetherifizierungen und Acylierungen auf Verbindungen gemäss Formel (I) ausgeführt, in denen freie Hydroxygruppen in $R_1$ vorhanden sind, und in denen aber im Teil X die terminale Carboxylgruppe durch Gruppen geschützt sind, die verschieden als die für X vorgesehene Amid- oder Estergruppen sind, und/oder in welchen eine oder mehrere der vorhandenen hydrophilen Gruppen in geschützter Form, z.B. wie oben beschrieben, vorliegen, so erhält man Ausgangsstoffe für die oben genannte Variante a).

In den nach irgend einer der beschriebenen Verfahrensvarianten erhaltenen Lipopeptiden gemäss Formel I, in welchen eine freie terminale Carboxylgruppe im Teil X vorhanden ist, kann dieselbe in an sich bekannter Weise, z.B. nach einer der in der Peptidchemie gebräuchlichen Methoden, in die Amid- oder Estergruppen übergeführt werden.

Von den Lipopeptiden gemäss Formel (I) und den im vorhergehenden beschriebenen Untergruppen derselben sind besonders diejenigen bevorzugt, die die R-Konfiguration am C*-Atom des Cysteinrestes besitzen.

Eine als Ausgangsstoff zu verwendende Verbindung XI, in welcher W = OH ist, kann durch Kondensation des substituierten Cysteins oder Homocysteins der Formel

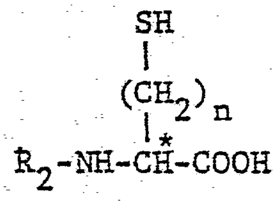

(XVII), n = 1,2 * = S/R oder S oder R

worin $R_2$ die gleiche Bedeutung wie in Formel I hat, in Gegenwart eines basischen Mittels mit einer Verbindung der Formel

$$R_1 - T \qquad (XVIII),$$

worin T eine reaktionsfähige, funktionell abgewandelte Hydroxygruppe bedeutet, erhalten werden. Vorzugsweise wird dabei die Cystein- bzw. Homocystein-carboxylgruppe durch eine in der Peptidchemie gebräuchliche Schutzgruppe intermediär geschützt.

Eine Kondensation gemäss obiger Methode f) wird ebenfalls in an sich bekannter Weise ausgeführt. Eine reaktionsfähige funktionell abgewandelte Hydroxygruppe T ist z.B. eine veresterte Hydroxygruppe, z.B. eine mit einer aliphatischen oder aromatischen Sulfonsäure, z.B. einer niederen aliphatischen Sulfonsäure mit 1-7 C-Atomen oder einer monocyclischen aromatischen Sulfonsäure, wie einer Benzolsulfonsäure. In erster Linie wird ein p-Toluolsulfonsäure-Ester als Verbindung gemäss Formel XV verwendet.

Eine reaktionsfähige veresterte Hydroxygruppe ist aber auch ein Halogenatom, wie z.B. Chlor, Brom oder Jod, oder insbesondere auch eine Epoxygruppe.

Ein zur Durchführung der Kondensation geeignetes basisches Medium ist eine schwach basische oder eine stark basische Verbindung, wie z.B. ein Alkalimetall- oder Erdalkalimetallhydrid oder Magnesiumhydrid, ein Alkalisalz einer schwachen Säure, wie Natrium- oder Kaliumcarbonate. Bei Verwendung von Halogenverbindungen zur Kondensation ist Silberoxid ein bevorzugtes basisches Medium. Im Falle eines Epoxids wird ebenfalls vorzugsweise ein Alkalimetall einer schwachen Säure als Kondensationsmittel verwendet, insbesondere Natrium- oder Kaliumcarbonat.

Bei der Acylierungsmethode gemäss der Variante g) wird der Ausgangsstoff der Formel XVI mit einem den Rest $R_2$ -CO-einführenden Acylierungsmittel behandelt, wobei $R_2$ die gleiche Bedeutung wie in Formel I hat. Die Bedingungen für diese Acylierung sind dieselben wie für die oben genannten Varianten d) und e). Insbesondere muss darauf geachtet werden, dass freie Aminogruppen in der Peptidkette X in Salzform übergeführt werden, z.B. ins Hydrochlorid oder in ein quaternäres Ammoniumsalz.

Die zur verfahrensgemässen Herstellung der neuen Lipopeptide zu verwendenden Aminosäuren bzw. Peptide sind bekannt oder können nach an sich bekannten Methoden hergestellt werden.

Die noch nicht erwähnten Ausgangsverbindungen zur Ausführung der oben genannten Verfahren a) bis g) können in an sich bekannter Weise hergestellt werden. Z.B. können Verbindungen der Formel XVI aus entsprechenden Verbindungen hergestellt werden, in denen die Aminogruppe geschützt ist, wobei die Schutzgruppen diejenigen, die aus der Peptidchemie bekannt sind, sein können. Diese Verbindungen können ihrerseits aus den entsprechenden geschützten Cysteinen durch Umsetzen mit einer Verbindung der Formel $R_1$ - T, wie oben beschrieben, erhalten werden.

Die gemäss Variante b) des oben angegebenen Verfahrens zu verwendenden Ausgangsstoffe der Formel XI, worin W eine Aminosäure oder eine zur vollständigen Aminosäurensequenz komplementäre Sequenz bedeutet, können nach analogen Methoden wie für die Varianten a) oder b) erhalten werden.

Die obigen Ausgangsverbindungen (XI) für die Variante b) für den Fall, dass W = OH ist, sowie ihre diastereomeren Gemische, Salze und Komplexe besitzen selber auch eine immunpotenzierende Wirkung, wobei diese in vitro im gleichen Dosisbereich wie für die Verbindungen der Formel I und ihre Derivate angegeben, auftritt. In vivo zeigen die genannten Verbindungen im oben beschriebenen Test zur Potenzierung der humoralen Immunität bei der intraperitonealen Verabreichung 5 Tage vor der Antigengabe einen Anstieg der Antikörpertiter im Serum im Dosisbereich von 1.0 bis 3 mg/kg Tier.

Die erhaltenen Lipopeptide können in an sich bekannter Weise in ihre Salze übergeführt werden, z.B. durch Umsetzen erhaltener saurer Verbindungen mit Alkali- oder Erdalkalihydroxyden oder erhaltener basischer Verbindungen mit Säuren.

Infolge der engen Beziehung zwischen den neuen Verbindungen in freier Form und in Form ihrer Salze und Komplexe sind im vorausgegangenen und nachfolgend unter den freien Verbindungen sinn- und zweckgemäss gegebenenfalls auch die entsprechenden Salze zu verstehen.

Erhaltene Isomerengemische können auf Grund der physikalisch-chemischen Unterschiede der Bestandteile in bekannter Weise aufgetrennt werden, beispielsweise durch Chromatographie und/oder fraktionierte Kristallisation. Vorteilhafterweise isoliert man das wirksamere der Isomeren.

Die oben beschriebenen Verfahren werden z.B. nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas, wie Stickstoffatmosphäre. Dabei sind unter Berücksichtigung aller im Molekül befindlichen Substituenten, wenn erforderlich, insbesondere bei Anwesenheit leicht hydrolysierbarer O-Acylreste, besonders schonende Reaktionsbedingungen, wie kurze Reaktionszeiten, Verwendung von milden sauren Mitteln in niedriger Konzentration stöchiometrische Mengenverhältnisse, Wahl geeigneter Katalysatoren, Lösungsmittel, Temperatur und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche die beschriebenen neuen Lipopeptide gemäss der Erfindung, sowohl solche entsprechend der Formel (I) wie solche der Formel (XI) ihre Gemische, Salze oder Komplexe enthalten. Bei den erfindungsgemässen pharmazeutischen Präparaten handelt es sich um solche zur enteralen, wie oralen oder rektalen, sowie parenteralen Verabreichung

an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, sowie von der Applikationsweise ab.

Zur Erzielung eines immunpotenzierenden Effektes an Mensch oder Tier werden z.B. tägliche Dosen im Bereich zwischen 1-30 mg/kg Körpergewicht Lipopeptid bei subcutaner Applikation und etwa im Bereiche von 0,03-3 mg/kg Körpergewicht bei intraperitonealer Applikationsart verabreicht.

Die neuen pharmazeutischen Präparate enthalten von etwa 10% bis etwa 95%, vorzugsweise von etwa 20% bis etwa 90% des Wirkstoffs. Erfindungemässe pharmazeutische Präparate können z.B. in Dosiseinheitsform, wie Dragées, Tabletten, Kapseln, Suppositorien oder Ampullen vorliegen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie

die obgenannten Stärken, ferner Carboxymethylstärke, quervernetztes Polyvinylpyrrolidon, Agar, Alginsäure oder ein Salz davon, wie Natriumalginat Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die nachfolgenden Beispiele illustrieren die oben beschriebenen Erfindung; sie sollen jedoch diese in ihrem Umfang in keiner Weise einschränken. Temperaturen werden in Celsiusgraden angegeben.

Die benützten Abkürzungen bedeuten:

Z = Carbobenzoxy

$Bu^t$ = tert. Butyläther

O $Bu^t$ = tert. Butylester

In DC verwendet man Kieselgel als Adsorbens und folgende Systeme als Laufmittel:

System 3: Essigester-Pyridin-Wasser (65:20:15)

System 157: Chloroform-Methanol-Wasser-Eisessig (70:42:10:0.5)

System 157c: Chloroform-Methanol-Wasser-Eisessig (75:25:5:0.5)

System 157 A Chloroform-Methanol-Wasser-Eisessig (90:10:1:0,5)

Beispiel 1: 1,25 g N-Palmitoyl-S-[2(R),3-dihexadecyloxy-propyl]-Cys-Ser($Bu^t$)-Ser($Bu^t$)-Phe-Ala-Glu($OBu^t$)$_2$ werden in 15 ml 90-prozentiger Trifluoressigsäure aufgenommen und die Lösung nach 60 Minuten bei 20° eingedampft. Der Rückstand wird in 5 ml Essigester digeriert und dann über Kaliumhydroxid getrocknet. Das so erhaltene Lipopeptid N-Palmitoyl-S-[2(R),3-dihexadecyloxypropyl]-Cys-Ser-Ser-Phe-Ala-Glu-OH zeigt im Dünnschichtchromatogramm auf Silicagel einen Rf-Wert von 0,32 (157 c).

Der obige Ausgangsstoff wird folgendermassen hergestellt: Zu 1.7 g N-Palmitoyl-S-[2(R), 3-dihexadecyloxy-propyl]-(L)-cystein und 1,47 g H-Ser($Bu^t$)-Ser($Bu^t$)-Phe-Ala-Glu-($OBu^t$)$_2$ in 25 ml Dimethylformamid werden 0,475 g Dicyclohexylcarbodiimid und 0,355 g N-Hydroxybenzotriazol gegeben. Nach 20 Stunden bei 20° wird das Lösungsmittel bei reduziertem Druck abgedampft und der Rückstand durch Chromatographie an einer Säule von Silicagel gereinigt. Chloroform-Methanol (99:1) eluiert das reine N-Palmitoyl-S-[2(R), 3-dihexadecyloxypropyl]-Cys-Ser($Bu^t$)-Ser ($Bu^t$)-Phe-Ala-Glu($OBu^t$)$_2$, dessen Rf-Wert im Dünnschichtchromatogramm auf Silicagel 0,63 (Chloroform-Methanol 95:5) beträgt.

Beispiel 2: 0,95 g N-Palmitoyl-S-[2(R,S)-hydroxy-octadecyl]-Cys-Ser($Bu^t$)-Ser($Bu^t$)-Phe-Ala-Glu($OBu^t$)$_2$ werden in 10 ml 90 prozentiger Trifluoressigsäure aufgenommen und die Lösung nach 45 Minuten bei 20° bei reduziertem Druck eingedampft. Der Rückstand wird in Wasser digeriert und dann über Kaliumhydroxid getrocknet. Man erhält das reine N-Palmitoyl-S-[2(R,S)-hydroxy-octadecyl]-Cys-Ser-Ser-Phe-Ala-Glu-OH, das im Dünnschichtchromatogramm auf Silicagel einen Rf-Wert von 0,35 (157) zeigt.

Der obige Ausgangsstoff wird analog zu Beispiel 1 durch Kondensation von N-Palmitoyl-S-[2(R,S)-hydroxy-octadecyl]-(L)-cystein mit H-Ser-($Bu^t$)-Ser($Bu^t$)-Phe-Ala-Glu($OBu^t$)$_2$ erhalten. Rf-Wert im Dünnschichtchromatogramm auf Silicagel: 0,13 (Chloroform-Methanol 98:2); 0,60 (157 A).

<u>Beispiel 3:</u> 1,1 g N-Palmitoyl-S-[2(R,S)-palmitoyloxy-octadecyl]-Cys-Ser($Bu^t$)-Ser($Bu^t$)-Phe-Ala-Glu($OBu^t$)$_2$ werden in 15 ml 90-prozentiger Trifluoressigsäure aufgenommen und nach 60 Minuten bei 20° eingedampft. Der Rückstand wird mit Essigester digeriert und dann über Kaliumhydroxid getrocknet. Es resultiert das N-Palmitoyl-S-[2(R,S)-palmitoyloxy-octadecyl]-Cys-Ser-Ser-Phe-Ala-Glu-OH, welches im Dünnschichtchromatogramm auf Silicagel einen Rf-Wert von 0,44 (157) zeigt.

Der Ausgangsstoff wird folgendermassen hergestellt:

Zu 1,3 g N-Palmitoyl-S-[2(R,S)-palmitoyloxy-octadecyl]-(L)-cystein und 1,5 g H-Ser($Bu^t$)-Ser($Bu^t$)-Phe-Ala-Glu($OBu^t$)$_2$ in 25 ml Dimethylformamid werden 0,32 g N-Hydroxybenzotriazol und 0,37 g Dicyclohexylcarbodiimid gegeben. Nach 15 Stunden bei Raumtemperatur wird das Lösungsmittel bei reduziertem Druck abgedampft und der Rückstand durch Chromatographie an einer Säule von Silicagel gereinigt. Chloroform-Methanol 98:2 eluieren das N-Palmitoyl-S-[2(R,S]-palmitoyloxy-octadecyl]-Cys-Ser-($Bu^t$)-Ser($Bu^t$)-Phe-Ala-Glu($OBu^t$)$_2$ mit dem Rf-Wert 0,48 (Chloroform-Methanol 95:5) und 0,74 (157 A) im Dünnschichtchromatogramm auf Silicagel.

<u>Beispiel 4:</u> 0,98 g N-Palmitoyl-S-octadecyl-Cys-Ser-($Bu^t$)-Ser($Bu^t$)-Phe-Ala-D-Glu($OBu^t$)$_2$ werden in 10 ml 90-prozentiger Trifluoressigsäure aufgenommen. Die Lösung

wird nach 50 Minuten bei 20° unter vermindertem Druck eingedampft und der Rückstand über Kaliumhydroxid getrocknet. Man erhält das N-Palmitoyl-S-octadecyl—Cys-Ser-Ser-Phe-Ala-D-Glu-OH mit dem Rf-Wert 0,43 (157 c) im Dünnschichtchromatogramm auf Silicagel.

Ausgangsstoffe können folgendermassen erhalten werden: 1,22 g N-Palmitoyl-S-octadecyl—(L)-cystein und 1,53 g H-Ser-($Bu^t$)-Ser($Bu^t$)-Phe-Ala-D-Glu($OBu^t$)$_2$ in 25 ml Dimethylformamid werden mit 0,5 g Dicyclohexylcarbodiimid und 0,37 g N-Hydroxybenzotriazol versetzt. Nach 20 Stunden wird das Reaktionsgemisch bei vermindertem Druck eingedampft und der Rückstand durch Chromatographie an einer Säule von Silicagel gereinigt. Durch Chloroform-Methanol (97:3) wird das N-Palmitoyl-S-octadecyl—Cys-Ser($Bu^t$)-Ser($Bu^t$)-Phe-Ala-D-Glu($OBu^t$)$_2$ eluiert. Rf-Wert im Dünnschichtchromatogramm auf Silicagel: 0,80 (Chloroform-Methanol 9:1).

1,84 g Z-Phe-Ala-D-Glu($OBu^t$)$_2$ werden in 50 ml Methanol gelöst und nach Zugabe von 0,2 g Pd-Kohle (10%-ig) bei 20° während 3 Stunden hydriert. Der Katalysator wird abfiltriert und das Filtrat eingedampft. Das erhaltene H-Phe-Ala-D-Glu($OBu^t$)$_2$ zeigt im Dünnschichtchromatogramm auf Silicagel einen Rf-Wert von 0,60 (Chloroform-Methanol 8:2).

2,79 g Z-Phe-OH und 3,08 g H-Ala-D-Glu($OBu^t$)$_2$ werden in 60 ml Dimethylformamid gelöst und bei 0° mit 2,31 g Dicyclohexylcarbodiimid und 1,57 g N-Hydroxybenzotriazol versetzt. Nach 20 Stunden bei Raumtemperatur filtriert man ab und dampft das Filtrat ein. Der Rückstand wird in 200 ml Essigester aufgenommen und die Lösung mit 1N Zitronensäure, 1N Natriumbicarbonat und Wasser extrahiert. Nach dem Trocknen der Lösung mit Natriumsulfat wird eingedampft und der Rückstand aus Methanol-Aether umgefällt. Man erhält Z-Phe-Ala-D-Glu$(OBu^t)_2$, dessen Rf-Wert im Dünnschichtchromatogramm auf Silicagel 0,80 beträgt (Chloroform-Methanol 8:2).

4,33 g Z-Ala-D-Glu$(OBu^t)_2$ löst man in 45 ml Methanol und hydriert nach Zugabe von 0,4 g Pd-Kohle (10%-ig) während einer Stunde bei Raumtemperatur. Es wird dann vom Katalysator abfiltriert und das Lösungsmittel abgedampft. Das als Rückstand erhaltene H-Ala-D-Glu$(OBu^t)_2$ zeigt im Dünnschichtchromatogramm auf Silicagel einen Rf-Wert von 0,24 (Chloroform-Methanol 9:1).

Zu 2,23 g Z-Ala-OH und 2,96 g HCl.H-D-Glu$(OBu^t)_2$ in 50 ml Dimethylformamid gibt man bei 0° 1,27 ml N-Aethylmorpholin, 2,48 g Dicyclohexylcarbodiimid und 1,68 g N-Hydroxybenzotriazol. Nach 15 Stunden bei 4° wird abfiltriert, das Filtrat eingedampft und der Rückstand in Essigester gelöst. Die Lösung wird mit 1 N Zitronensäure, 1 N-Natriumbicarbonat und Wasser extrahiert und dann über Natriumsulfat getrocknet. Nach Abdampfen des Lösungsmittels wird das erhaltene Z-Ala-D-Glu$(OBu^t)_2$ aus Essigester-Petroläther umgefällt. Rf-Wert im Dünnschichtchromatogramm auf Silicagel: 0,72 (Chloroform-Methanol 9:1).

1,36 g Z-Ser$(Bu^t)$-Ser$(Bu^t)$-NH-$NH_2$ werden in 15 ml Dimethylformamid gelöst und bei -15° mit 3,29 ml 2,28 N HCl in Essigester und 0,381 ml tert.Butylnitrit versetzt. Nach 10 Minuten bei -10° gibt man eine Lösung von 1,44 g H-Phe-Ala-D-Glu$(OBu^t)_2$ und 1,51 ml N-Aethylmorpholin in 10 ml Dimethylformamid zu und lässt eine Stunde bei -10° und 15 Stunden bei 0° reagieren. Die

Lösung wird dann eingedampft, der Rückstand in Essigester aufgenommen und diese Lösung mit 1N Zitronensäure, 1N Natriumbicarbonat und Wasser extrahiert, über Natriumsulfat getrocknet und eingedampft. Das erhaltene Z-Ser-$(Bu^t)$-Ser$(Bu^t)$-Phe-Ala-D-Glu$(OBu^t)_2$ wird aus Essigester-Hexan umkristallisiert. Smp. 205-209°. $[\alpha]_D$ = -4°(c=0,6 in $CHCl_3$).

2,3 g dieses Produktes werden in 40 ml Methanol gelöst und nach Zugabe von 0,5 g Pd-Kohle (10%-ig) bei 20° während 2 Stunden hydriert. Der Katalysator wird abfiltriert und das Filtrat eingedampft, wobei das H-Ser$(Bu^t)$-Ser$(Bu^t)$-Phe-Ala-D-Glu$(OBu^t)_2$ als weisser Schaum anfällt. Rf = 0.47 in Chloroform-Methanol 9:1.

In analoger Weise wird H-Ser-$(Bu^t)$-Ser$(Bu^t)$-Asn-Ala-D-Glu $(OBu^t)_2$ hergestellt. Rf-Wert im Dünnschichtchromatogramm auf Silicagel: 0,48 (Chloroform-Methanol 8:2).

Z-Ser$(Bu^t)$-Ser$(Bu^t)$-Asn-Ala-D-Glu$(OBu^t)_2$: Smp. 168-170°. $[\alpha]_D$ = -6°(c=0,8 in $CH_3OH$).

<u>Beispiel 5</u>: 1,4 g N-Palmitoyl-S-[2(R,S),3-dihexadecyloxypropyl]-(D,L)homocysteinyl-Ser$(Bu^t)$-Ser$(Bu^t)$-Asn-Ala-Glu$(OBu^t)_2$ werden in 15 ml 90-prozentiger Trifluoressigsäure aufgenommen und nach 50 Minuten bei 20° eingedampft. Der Rückstand wird über Kaliumhydroxid getrocknet. N-Plamitoyl-S-[2(R,S),3-dihexadecyloxypropyl]-(D,L)homocysteinyl-Ser-Asn-Ala-Glu-OH fällt als wächsernes Produkt an.

Der Ausgangsstoff kann nach der in den Beispielen 1 bis 4 beschriebenen Methodik aus N-Palmitoyl-S-[2(R,S),3-dihexadecyloxypropyl]-(D,L)homocystein und H-Ser$(Bu^t)$-Ser-$(Bu^t)$-Asn-Ala-Glu$(OBu^t)_2$ gewonnen werden.

<u>Beispiel 6</u>: 1,2 g N,S-Dipalmitoyl-(L)-Cys-Ser$(Bu^t)$-Ser$(Bu^t)$-Phe-Ala-Glu$(OBu^t)_2$ werden in 15 ml 90-prozentiger Trifluoressigsäure während 45 Minuten deblockiert. Eindampfen der Trifluoressigsäure liefert N,S-Dipalmitoyl-Cys-Ser-Ser-Phen-Ala-Glu-OH.

Der Ausgangsstoff wird wie in den Beispielen 1 bis 4 aus N,S-Dipalmitoyl-(L)-cystein und H-Ser($Bu^t$)-Ser($Bu^t$)-Phe-Ala-Glu($OBu^t$)$_2$ erhalten.

Beispiel 7: Geht man von N-Palitoyl-S-[2(R), 3-dihexadecyloxypropyl]-(L)-cystein, N-Palmitoyl-S-[2(R,S)-hydroxy-octadecyl]-(L)-cystein, N-Palmitoyl-S-[2(R,S)-palmitoyloxy-octadecyl]-(L)-cystein, N-Palmitoyl-S-octadecyl-(L)-cystein, N-Palmitoyl-S-[2(R,S),3-dihexadecyloxypropyl]-(D,L)-homocystein, N,S-Dipalmitoyl-L-cystein, N-Palmitoyl-S-[2(R,S)-palmitoyloxy-eicosanyl]-(L)-cystein aus, die im Folgenden in dieser Reihenfolge genommen als die Verbindungen $C_1OH$, $C_2OH$, $C_3OH$, $C_4OH$, $C_5OH$, $C_6OH$, $C_7OH$ bezeichnet werden, wobei OH die Hydroxylgruppe der Carboxylfunktion des substituierten Cysteins bedeutet, und kondensiert jede dieser Verbindungen mit den nachstehenden Peptiden unter Anwendung der gleichen Schutzgruppen wie in den Beispielen 1 bis 4, und nach derselben Methodik,

H-Ser-Ser-D-Asn-Ala-Glu-OH
H-Thr-Thr-Asn-Ala-Lys-OH
H-Ser-Thr-Asn-D-Ala-Lys-OH
H-Ser-Ser-Asn-Ala-Glu-OH
H-Thr-Ser-Phe-Ala-D-Glu-OH
H-Ser-Ser-Phe-Ala-D-Glu-OH
H-Ser-Ser-Phe-Ala-Glu-OH
H-D-Ser-D-Ser-D-Phe-Ala-D-Glu-OH
H-D-Thr-D-Phe-D-Ala-D-Glu-OH

so erhält man die folgenden Lipopeptide:

$C_1$-Ser-Ser-D-Asn-Ala-Glu-OH
$C_1$-Thr-Thr-Asn-Ala-Lys-OH
$C_1$-Ser-Thr-Asn-D-Ala-Lys-OH
$C_1$-Ser-Ser-Asn-Ala-Glu-OH
$C_1$-Thr-Ser-Phe-Ala-D-Glu-OH
$C_1$-Ser-Ser-Phe-Ala-D-Glu-OH
$C_1$-Ser-Ser-Phe-Ala-Glu-OH
$C_1$-D-Ser-D-Ser-D-Phe-Ala-D-Glu-OH
$C_1$-D-Thr-D-Phe-D-Ala-D-Glu-OH

$C_2$-Ser-Ser-D-Asn-Ala-Glu-OH
$C_2$-Thr-Thr-Asn-Ala-Lys-OH
$C_2$-Ser-Thr-Asn-D-Ala-Lys-OH
$C_2$-Ser-Ser-Asn-Ala-Glu-OH
$C_2$-Thr-Ser-Phe-Ala-D-Glu-OH
$C_2$-Ser-Ser-Phe-Ala-D-Glu-OH
$C_2$-Ser-Ser-Phe-Ala-Glu-OH
$C_2$-D-Ser-D-Ser-D-Phe-Ala-D-Glu-OH
$C_2$-D-Thr-D-Phe-D-Ala-D-Glu-OH

$C_3$-Ser-Ser-D-Asn-Ala-Glu-OH
$C_3$-Thr-Thr-Asn-Ala-Lys-OH
$C_3$-Ser-Thr-Asn-D-Ala-Lys-OH
$C_3$-Ser-Ser-Asn-Ala-Glu-OH
$C_3$-Thr-Ser-Phe-Ala-D-Glu-OH
$C_3$-Ser-Ser-Phe-Ala-D-Glu-OH
$C_3$-Ser-Ser-Phe-Ala-Glu-OH
$C_3$-D-Ser-D-Ser-D-Phe-Ala-D-Glu-OH
$C_3$-D-Thr-D-Phe-D-Ala-D-Glu-OH

$C_4$-Ser-Ser-D-Asn-Ala-Glu-OH
$C_4$-Thr-Thr-Asn-Ala-Lys-OH
$C_4$-Ser-Thr-Asn-D-Ala-Lys-OH
$C_4$-Ser-Ser-Asn-Ala-Glu-OH
$C_4$-Thr-Ser-Phe-Ala-D-Glu-OH

$C_4$-Ser-Ser-Phe-Ala-D-Glu-OH
$C_4$-Ser-Ser-Phe-Ala-Glu-OH
$C_4$-D-Ser-D-Ser-D-Phe-Ala-D-Glu-OH
$C_4$-D-Thr-D-Phe-D-Ala-D-Glu-OH

$C_5$-Ser-Ser-D-Asn-Ala-Glu-OH
$C_5$-Thr-Thr-Asn-Ala-Lys-OH
$C_5$-Ser-Thr-Asn-D-Ala-Lys-OH
$C_5$-Ser-Ser-Asn-Ala-Glu-OH
$C_5$-Thr-Ser-Phe-Ala-D-Glu-OH
$C_5$-Ser-Ser-Phe-Ala-D-Glu-OH
$C_5$-Ser-Ser-Phe-Ala-Glu-OH
$C_5$-D-Ser-D-Ser-D-Phe-Ala-D-Glu-OH
$C_5$-D-Thr-D-Phe-D-Ala-D-Glu-OH

$C_6$-Ser-Ser-D-Asn-Ala-Glu-OH
$C_6$-Thr-Thr-Asn-Ala-Lys-OH
$C_6$-Ser-Thr-Asn-D-Ala-Lys-OH
$C_6$-Ser-Ser-Asn-Ala-Glu-OH
$C_6$-Thr-Ser-Phe-Ala-D-Glu-OH
$C_6$-Ser-Ser-Phe-Ala-D-Glu-OH
$C_6$-Ser-Ser-Phe-Ala-Glu-OH
$C_6$-D-Ser-D-Ser-D-Phe-Ala-D-Glu-OH
$C_6$-D-Thr-D-Phe-D-Ala-D-Glu-OH

$C_7$-Ser-Ser-D-Asn-Ala-Glu-OH
$C_7$-Thr-Thr-Asn-Ala-Lys-OH
$C_7$-Ser-Thr-Asn-D-Ala-Lys-OH
$C_7$-Ser-Ser-Asn-Ala-Glu-OH
$C_7$-Thr-Ser-Phe-Ala-D-Glu-OH
$C_7$-Ser-Ser-Phe-Ala-D-Glu-OH
$C_7$-Ser-Ser-Phe-Ala-Glu-OH
$C_7$-D-Ser-D-Ser-D-Phe-Ala-D-Glu-OH
$C_7$-D-Thr-D-Phe-D-Ala-D-Glu-OH

Beispiel 8: Die substituierten Cysteine, die in den vorhergehenden oder nachstehenden Beispielen als Ausgangsstoffe eingesetzt werden, können z.B. folgendermassen erhalten werden:

189 mg N-Palmitoyl-(L)-cystein gibt man zu 41,5 mg Natriumhydrid-Dispersion in etwa 5 ml absolutem Dioxan und lässt bei 0° unter Stickstoff bis zur beendeten Wasserstoffentwicklung ausreagieren. Man gibt 330 mg 1-Tosyl-2(R),3-dihexadecyl-glycerin in 2 ml Dioxan zu und erhitzt 15 Stunden auf 90°. Danach dampft man ein, säuert mit 1 N HCl auf pH ca. 3 an und verteilt zwischen Chloroform und Wasser. Die Chloroform-Phase wird getrocknet und eingedampft und der Rückstand an 9 g Silicagel Merck in Chloroform/Aceton 8:2 gereinigt. Man erhält nach Umkristallisieren aus Essigester farblose Kristalle vom Smp. 65-66°, $[\alpha]_D^{20} = + 3° \pm 1°$ (Chloroform, c = 0,53), des N-Palmitoyl-S-[2(R),3-dihexadecyloxypropyl]-(L)-cysteins.

In analoger Weise erhält man aus 1-Tosyl-2(S,R), 3-dihexadecylglycerin das N-Palmitoyl-S[-2(S,R),3-dihexadecyloxy-propyl]-(L)-cystein vom Smp. 87-88° $[\alpha]_D^{20} = + 6° \pm 1°$ (c =0.45 in Chloroform).

5,6 g N-Palmitoylcystein, 5 g 1,2-Epoxy-octadecen und 5,35 g Pottasche in 100 ml Aethanol erhitzt man unter Stickstoff 15 Stunden auf 80°. Man dampft ein, säuert mit 1 N HCl auf pH ca. 3 an und schüttelt mit Chloroform aus, wäscht die Chloroform-Phase mit Wasser und trocknet sie mit Natriumsulfat. Nach dem Eindampfen erhält man einen farblosen Rückstand, der aus Essigester umkristallisiert wird. Man erhält farblose Kristalle des N-Palmitoyl-S-[2(S,R)-hydroxy-octadecyl]-(L)-cysteins vom Smp. 96-98° $[\alpha]_D^{20} = + 16°$ (c = 0,78, $CHCl_3$).

In analoger Weise erhält man aus N-Palmitoyl-(L)-cystein und 1,2-Epoxy-dodecen das N-Palmitoyl-S-[2(S,R)-hydroxy-dodecyl]-(L)-cystein vom Smp. 79-80° $[\alpha]_D^{20}$ = +19° (c = 0,752, $CHCl_3$) und aus N-Palmitoylcystein und 1,2-Epoxy-eicosen das N-Palmitoyl-S-[2 (S,R)-hydroxy-eicosanyl]-(L)-cystein vom Smp. 94-95°, $[\alpha]_D^{20}$ = +14° (c=0,655, $CHCl_3$).

0,2 g N-Palmitoyl-S-[ 2(R,S)-palmitoyloxyoctadecyl]-(L)-cystein-benzhydrylester löst man bei Raumtemperatur in einer Mischung aus 0,4 ml Trifluoressigsäure und 1,6 ml Methylenchlorid und lässt 2 Stunden reagieren. Man dampft im Vakuum zur Trockene ein, löst den Rückstand in Chloroform und schüttelt die Chloroform-Phase mehrmals mit Wasser aus. Nach Trocknen mit Natriumsulfat und Eindampfen erhält man einen kristallinen Rückstand, den man in 2 ml heissem Petroläther löst; nach Zugabe von 2 ml Ligroin kühlt man auf -10° ab und erhält Kristalle des N-Palmitoyloxy-S-[2-(R,S)-palmitoyloxyoctadecyl]-(L)-cysteins vom Smp. 80-83°.

Den Ausgangsstoff erhält man wie folgt: 0,8 g N-Palmitoyl-S-[2(R,S)-hydroxy-octadecyl]-(L)-cystein-benzhydrylester in 5 ml Pyridin versetzt man mit 0,56 ml Palmitinsäurechlorid in 5 ml Methylenchlorid. Nach 24 Stunden bei Raumtemperatur ist die Acylierung nach Dünnschichtchromatogramm (Silicagel Merck, Chloroform) beendet. Man gibt zur Reaktionslösung 2 ml Methanol und dampft nach 20 Minuten im Vakuum ein. Man nimmt den Rückstand in Chloroform auf und schüttelt je zweimal mit 1 N HCl, gesättigter Natriumbicarbonat-Lösung und Wasser aus, trocknet die Chloroform-Phase über Natriumsulfat und dampft ein. Nach Reinigung des Rückstandes über 20 g Silicagel Merck mit Chloroform als Laufmittel erhält man farblose Kristalle, die sich aus Aceton/Methanol umkristallisieren lassen und die bei 46-47° schmelzen. Sie stellen den

N-Palmitoyl-S-[2(R,S)-palmitoyloxyoctadecyl]-(L)-cystein-benzhydrylester dar.

Den für die obige Umsetzung benötigten Benzhydrylester erhält man auf folgende Weise:

5,2 g N-Palmitoyl-S-[2(R,S)-hydroxy-octadecyl]-(L)-cystein löst man in 60 ml Chloroform/Aethanol 1:1-Gemisch und gibt dazu eine Lösung von 1,93 g Diphenyldiazomethan in 5 ml Chloroform. Nach 12 Stunden bei Raumtemperatur dampft man zur Trockene ein, verreibt den Rückstand mit Petroläther, dann mit Ligroin und kristallisiert ihn aus Ligroin um. Man erhält so farblose Kristalle des genannten Esters vom Smp. 93-95°.

In analoger Weise wie oben beschrieben kann man die folgenden substituierten Cysteine herstellen:

N-Palmitoyl-S-[2(R,S)-hydroxy-dodecyl]-(L)-cystein
N-Palmitoyl-S-[2(R,S)-palmitoyloxy-dodecyl]-(L)-cystein
N-Palmitoyl-S-[2(R,S)-hydroxy-eicosanyl]-(L)-cystein
N-Palmitoyl-S-[2(R,S)-palmitoyloxy-eicosanyl]-(L)-cystein

3,6 g N-Palmitoyl-(L)-cystein in 50 ml Aethanol versetzt man mit 4,8 g Docosanyl-p-toluolsulfonat und 5 g Pottasche und erhitzt unter Rühren und unter Stickstoff 20 Stunden auf 80°. Man dampft zur Trockene, säuert mit 1 N HCl bis pH = 3 an und verteilt zwischen Chloroform und Wasser. Nach Trocknen und Eindampfen der Chloroform-Phase erhält man einen farblosen kristallinen Rückstand, der aus Essigester umkristallisiert wird. Man erhält so das N-Palmitoyl-S-docosanyl-(L)-cystein.

In analoger Weise setzt man N-Palmitoyl-(L)-cystein mit 1-Brom-octadecan um und erhält das N-Palmitoyl-S-octadecyl-(L)-cystein vom Smp. 90-92°; $[\alpha]_D^{20} = +13°$ (c=1,33, $CHCl_3$).

In gleicher Weise erhält man das N-Palmitoyl-S-dodecyl-(L)-cystein.

Aus N-Palmitoyl-(L)-cystein und Cholesteryl-p-toluolsulfonat erhält man analog das N-Palmitoyl-S-[cholesteryl-(3)]-(L)-cystein.

Aus 1-(p-Toluolsulfonyl)-di-äthylenglykol-monocetyläther und N-Palmitoyl-(L)-cystein erhält man das N-Palmitoyl-S-(3,6-dioxa-docosanyl)-(L)-cystein.

Aus dem p-Toluolsulfonat von Retinol (Vitamin-A-Alkohol) erhält man analog N-Palmitoyl-S-retinyl-(L)-cystein, und aus dem p-Toluolsulfonat vom Phytol erhält man analog N-Palmitoyl-S-phytyl-(L)-cystein.

Aus Farnesylbromid erhält man N-Palmitoyl-S-farnesyl-(L)-cystein.

Beispiel 9: Folgende substituierte Cysteine

$Cys_1$ = N-Palmitoyl-S-[2(R,S),3-dihexadecyloxy-propyl]-(L)-cystein
$Cys_2$ = N-Palmitoyl-S-[2(R,S)-hydroxydodecyl]-(L)-cystein
$Cys_3$ = N-Palmitoyl-S-2-(R,S)-hydroxyeicosanyl-(L)-cystein
$Cys_4$ = N-Palmitoyl-S-[2(R,S)-hydroxydodecyl]-(L)-cystein
$Cys_5$ = N-Palmitoyl-S-[2(R,S)-palmitoyloxydodecyl]-(L)-cystein
$Cys_6$ = N-Palmitoyl-S-[2(R,S)-hydroxy-eicosanyl]-(L)-cystein
$Cys_7$ = N-Palmitoyl-S-[2(R,S)-palmitoyloxy]-eicosanyl-(L)-cystein
$Cys_8$ = N-Palmitoyl-S-docosanyl-(L)-cystein
$Cys_9$ = N-Palmitoyl-S-octadecyl-(L)-cystein
$Cys_{10}$ = N-Palmitoyl-S-dodecyl-(L)-cystein

$Cys_{11}$ = N-Palmitoyl-S- [cholesteryl-(3)]-(L)-cystein
$Cys_{12}$ = N-Palmitoyl-S-(3,6-dioxa-docosanyl)-(L)-cystein
$Cys_{13}$ = N-Palmitoyl-S-retinyl-(L)-cystein
$Cys_{14}$ = N-Palmitoyl-S-phytyl-(L)-cystein
$Cys_{15}$ = N-Palmitoyl-S-farnesyl-(L)-cystein
werden gemäss den Angaben des Beispiels 5 mit jedem der folgenden Peptiden kondensiert:

H-Ser-Ser-Phe-Ala-Glu-OH
H-Ser-Ser-D-Asn-Ala-Glu-OH
H-Thr-Thr-Asn-Ala-Lys-OH
H-Ser-Thr-Asn-D-Ala-Lys-OH
H-Ser-Ser-Asn-Ala-Glu-OH
H-Thr-Ser-Phe-Ala-D-Glu-OH
H-Ser-Ser-Phe-Ala-D-Glu-OH

und man erhält so die entsprechenden Lipopeptide der allgemeinen Formel

$$Cys_n - X \text{ , wo}$$

X einer der eben angeführten Peptidsequenzen bedeutet, und $Cys_n$ eine beliebige der obigen substituierten Cystein-Derivate von $Cys_1$ - $Cys_{14}$ bedeutet.

Beispiel 10: N-Palmitoyl-S-[2(R,S), 3-dihexadecyloxy-propyl]-(L,D)-homo-cystein.

15,3 g L,D-Homocystein-thiolacton-hydrochlorid löst man in 150 ml Pyridin und versetzt tropfenweise unter Rühren mit 30 g Palmitinsäurechlorid in 50 ml Methylenchlorid. Nach 15-stündigem Rühren bei Raumtemperatur engt man im Vakuum ein, nimmt mit Chloroform auf und schüttelt mit 2 N Salzsäure, dann mit Wasser aus. Nach Trocknen der $CHCl_3$-Phase mit $Na_2SO_4$ und Einengen verreibt man den Rückstand mit Hexan und erhält Kristalle des N-Palmitoyl-(L,D)-homo-cystein-thiolactons.

5 g dieses Lactons versetzt man in 50 ml Aethanol unter Stickstoff mit 0,95 g (1 Aequivalent) KOH (85%) und 5 g Pottasche und erwärmt auf 70°. Nach 30 Minuten bei dieser Temperatur gibt man 10 g (1,04 Aequivalent) 1-Tosyl-(2,3-dihexadecyl)-glycerin in 20 ml Aethanol zu und lässt 20 Stunden bei 80° reagieren. Dann dampft man zur Trockne, säuert mit 1 N Salzsäure an, verteilt zwischen Chloroform und Wasser, trocknet die $CHCl_3$-Phase mit $Na_2SO_4$ und erhält nach Eindampfen einen Sirup, der über Kieselgel Merck in Chloroform: Aceton = 1:1 gereinigt wird. Man erhält nach Umkristallisieren aus Essigester farblose Kristalle.

N,S.-Dipalmitoyl-(L)-Cystein.

10,5 g L-Cystein-hydrochlorid-hydrat suspendiert man in einer Mischung aus 100 ml Pyridin und 50 ml Dimethylacetamid. Unter einer $N_2$-Atmosphäre und gutem Rühren gibt man eine Lösung von 17 g (18,7 ml) Palmitinsäurechlorid in 50 ml Methylenchlorid tropfenweise zu. Nach 25 Stunden bei Raumtemperatur dampft man die Reaktionslösung im Vakuum zum Sirup ein, säuert mit 2 N Salzsäure an und verteilt zwischen Chloroform und Wasser; man wäscht die $CHCl_3$-Phase, bis die Salzsäure entfernt ist. Nach Eindampfen der getrockneten $CHCl_3$-Phase erhält man einen Sirup, der beim Verreiben mit Hexan kristallisiert. Nach zweimaligem Umkristallisieren aus Aceton erhält man farblose Kristalle des N,S.-Dipalmitoyl-(L)-cysteins vom Smp. 82-84°.

Beispiel 11: 1,5 g N,S-Dipalmitoyl—Cys-Ser($Bu^t$)-Ser($Bu^t$)-Asn-Ala-Pro-$NH_2$ werden in 15 ml 90-prozentiger Trifluoressigsäure gelöst und die Lösung nach 45 Minuten bei Raumtemperatur eingedampft. Der Rückstand wird über Kaliumhydroxid getrocknet. Das so erhaltene N,S-Dipalmitoyl—Cys-Ser-Ser-Asn-Ala-Pro-$NH_2$ stellt ein weisses Pulver dar und besitzt im Dünnschichtchromatogramm auf Silicagel den Rf-Wert 0,50 (157 c).

Der Ausgangsstoff kann nach der in den Beispielen 1 bis 4 beschriebenen Methodik aus N,S-Dipalmitoyl—(L)-cystein und H-Ser($Bu^t$)-Ser($Bu^t$)-Asn-Ala-Pro-$NH_2$ gewonnen werden. Der Rf-Wert im Dünnschichtchromatogramm (Chloroform-Methanol 9:1) ist 0,20.

H-Ser($Bu^t$)-Ser($Bu^t$)-Asn-Ala-Pro-$NH_2$ [Rf-Wert im Dünnschichtchromatogramm auf Silicagel (157 c) = 0,53] kann in analoger Weise wie H-Ser($Bu^t$)-Ser($Bu^t$)-Phe-Ala-D-Glu($OBu^t$)$_2$ in Beispiel 4 hergestellt werden.

Beispiel 12: Nach der in Beispiel 11 beschriebenen Methode werden N,S-Dipalmitoyl—(L)-cystein und H-Ser($Bu^t$)-Ser($Bu^t$)-Asn-Ala-Val-$OCH_3$ kondensiert und man erhält so das N,S-Dipalmitoyl -Cys-Ser($Bu^t$)-Ser($Bu^t$)-Asn-Ala-Val-$OCH_3$ [Rf auf Silicagel mit Chloroform-Methanol 9:1 = 0,53]. 0,9 g dieses Stoffes werden in 10 ml 90-prozentiger Trifluoressigsäure aufgenommen und nach 45 Minuten wird die Lösung eingedampft. Der Rückstand wird mit Petroläther digeriert und dann über Kaliumhydroxid getrocknet. Man erhält das N,S-Dipalmitoyl—Cys-Ser-Ser-Asn-Ala-Val-$OCH_3$ als weisses Pulver. Rf-Wert im Dünnschichtchromatogramm auf Silicagel = 0,60 (157 c).

H-Ser(Bu$^t$)-Ser(Bu$^t$)-Asn-Ala-Val-OCH$_3$ kann analog der Herstellung von H-Ser(Bu$^t$)-Ser(Bu$^t$)-Phe-Ala-D-Glu(OBu$^t$)$_2$ in Beispiel 4 erhalten werden. [Rf-Wert im Dünnschichtchromatogramm auf Silicagel = 0,43 (157 c)].

Beispiel 13: 1.2 g N-Palmitoyl-S-octadecyl—D-Cys-Ser(Bu$^t$)-Ser(Bu$^t$)-Phe-Ala-Glu(OBu$^t$)$_2$ werden in 12 ml 90-prozentiger Trifluoressigsäure gelöst und die Lösung nach 45 Minuten Stehen bei Raumtemperatur eingedampft. Der Rückstand wird mit Petroläther digeriert und über Kaliumhydroxid getrocknet. Man erhält N-Palmitoyl-S-octadecyl—D-Cys-Ser-Ser-Phe-Ala-Glu-OH als weisses Pulver. Rf-Wert im Dünnschichtchromatogramm auf Silicagel 0.38 (157 c).

Der Ausgangsstoff kann nach der in den Beispielen 1 bis 4 beschriebenen Methode aus N-Palmitoyl-S-octadecyl—D-cystein und H-Ser(Bu$^t$)-Ser(Bu$^t$)-Phe-Ala-Glu(OBu$^t$)$_2$ gewonnen werden. Rf-Wert im Dünnschichtchromatogramm 0,70 (157 a).

Beispiel 14: 0,95 g N-Palmitoyl-S-octadecyl—(L,D)-homocysteinyl-Ser(Bu$^t$)-Ser(Bu$^t$)-Phe-Ala-Glu(OBu$^t$)$_2$ werden in 10 ml 90-prozentiger Trifluoressigsäure aufgenommen und die Lösung nach 40 Minuten eingedampft. Der Rückstand wird in Essigester digeriert und über Kaliumhydroxid getrocknet. N-Palmitoyl-S-octadecyl—(L,D)-homocysteinyl-Ser-Ser-Phe-Ala-Glu-OH fällt als weisses Pulver an. Rf-Wert im Dünnschichtchromatogramm auf Silicagel = 0,42 (157 c).

Die Ausgangsverbindung kann nach der in den Beispielen 1 bis 4 beschriebenen Methodik aus N-Palmitoyl-S-octadecyl—(L,D)-homocystein und H-Ser(Bu$^t$)-Ser(Bu$^t$)-Phe-Ala-Glu(OBu$^t$)$_2$ erhalten werden. Rf-Wert im Dünnschichtchromatogramm auf Silicagel = 0,75 (157 a).

Beispiel 15: 1,1 g N-Palmitoyl-S-farnesyl—Cys-Ser(Bu$^t$)-Ser(Bu$^t$)-Phe-Ala-Glu(OBu$^t$)$_2$ werden in 10 ml 90-prozentiger Trifluoressigsäure gelöst und die Lösung nach 40 Minuten zur Trockne eingedampft. Der Rückstand wird in Essigester digeriert und über Kaliumhydroxid getrocknet. N-Palmitoyl-S-farnesyl—Cys-Ser-Ser-Phe-Ala-Glu-OH fällt als weisses Pulver an. Rf-Wert im Dünnschichtchromatogramm auf Silicagel 0,35 (157 c).

Die Ausgangsverbindung kann nach der in den Beispielen 1 bis 4 beschriebenen Methodik aus N-Palmitoyl-S-farnesyl—(L)-cystein und H-Ser(Bu$^t$)-Ser(Bu$^t$)-Phe-Ala-Glu(OBu$^t$)$_2$ gewonnen werden.

Beispiel 16: Geht man von den in den Beispielen 13 bis 15 genannten N-Palmitoyl-S-octadecyl—(D)-cystein, N-Palmitoyl-S-octadecyl-(L,D)-homocystein und N-Palmitoyl-S-farnesyl-(L)-cystein aus, die im Folgenden in dieser Reihenfolge genommen, als die Verbindungen $C_8OH$, $C_9OH$ und $C_{10}OH$ bezeichnet werden, wobei OH die Hydroxylgruppe der freien Carboxylfunktion bedeutet, und kondensiert jede dieser Verbindungen mit den nachstehenden Peptiden unter Anwendung der gleichen Schutzgruppen wie in den Beispielen 1 bis 4 und nach derselben Methode,

H-Ser-Ser-Asn-Ala-Glu-OH
H-Ser-Ser-Asn-Ala-D-Glu-OH
H-Thr-Ser-Phe-Ala-L-Glu-OH
H-Ser-Ser-D-Phe-Ala-Glu-OH

so erhält man die folgenden Lipopeptide:

$C_8$-Ser-Ser-Asn-Ala-Glu-OH
$C_8$-Ser-Ser-Asn-Ala-D-Glu-OH
$C_8$-Thr-Ser-Phe-Ala-Glu-OH
$C_8$-Ser-Ser-D-Phe-Ala-Glu-OH

$C_9$-Ser-Ser-Asn-Ala-Glu-OH
$C_9$-Ser-Ser-Asn-Ala-D-Glu-OH
$C_9$-Thr-Ser-Phe-Ala-Glu-OH
$C_9$-Ser-Ser-D-Phe-Ala-Glu-OH

$C_{10}$-Ser-Ser-Asn-Ala-Glu-OH
$C_{10}$-Ser-Ser-Asn-Ala-D-Glu-OH
$C_{10}$-Thr-Ser-Phe-Ala-Glu-OH
$C_{10}$-Ser-Ser-D-Phe-Ala-Glu-OH.

Beispiel 17: 5 g (13,9 mMol) N-Palmitoyl-(L)-cystein und 4,76 g (16,7 mMol - 1,2 Aequivalente) Farnesylbromid löst man in 50 ml Aethanol, gibt nach Abkühlen auf 25° 4,8 g Pottasche hinzu und erhitzt 9 Stunden auf ca. 60° unter Stickstoff; dann gibt man noch 5 ml Aethanol zu und erhitzt weitere 6 Stunden auf 70°. Man dampft sodann im Vakuum zur Trockne ein, säuert mit 2N Salzsäure an und versetzt mit Essigester und Wasser. Man schüttelt zur Entfernung der Mineralsäure die Essigesterphase mehrmals mit Wasser aus. Nach Trocknen und Eindampfen der organischen Phase erhält man 8,1 g Syrup, den man über Kieselgel (Merck) mit Methylenchlorid-Aethanol 9:1 reinigt. Man erhält eine gelbliche, amorphe Substanz vom Rf-Wert 0,48 (Chloroform-Aethanol 9:1, auf Dünnschichtplatten Kieselgel Merck) und mit $[\alpha]_D^{20} = +2°$ (Chloroform, c = 0,435). Sie stellt das N-Palmitoyl-S-farnesyl—(L)-cystein dar.

Das N-Palmitoyl-S-octadecyl—(D)-cystein kann man folgendermassen herstellen:

Man gibt eine Lösung von 360 mg (1 mMol) N-Palmitoyl-(D)-cystein in 2 ml Dimethoxyäthan zu 48 mg Natriumhydrid-Dispersion (Mineralöl) in 1 ml Dimethoxyäthan unter Stickstoff. Nach 20-minütigem Rühren gibt man zur Suspension 400 mg 1-Brom-octadecan (1.2 mMol) in 4 ml Dimethoxyäthan. Man hält 15 Stunden bei 80° unter gutem Rühren und unter Stickstoffatmosphäre. Dann dampft man zur Trockne ein, nimmt mit Chloroform auf und säuert mit 1N Salzsäure an. Nach Zugabe von Chloroform und wässriger Salzsäure schüttelt man die Chloroform-Phase mehrmals mit Wasser aus, trocknet sie mit Natriumsulfat und dampft sie im Vakuum ein. Man erhält 600 mg eines leicht gelblichen kristallinen Pulvers, das in Chloroform-Aethanol 9:1 über Silicagel gereinigt wird. Man erhält farblose Kristalle des N-Palmitoyl-S-octadeyl-(D)-cysteins vom Rf-Wert 0,55 (Dünnschichtplatten Silicagel Merck, Chloroform-Aethanol 9:1) mit dem Smp. 100-102°, $[\alpha]_D^{20}$ = -12° (Chloroform).

N-Palmitoyl-S-octadecyl-(L,D)-homocystein kann auf folgende Weise erhalten werden:

Zu 3,55 g (0,01 Mol) N-Palmitoyl-(L,D)-homocysteinthiolacton in 40 ml Aethanol-Dioxan 2:1 gibt man 5,1 ml 4N Natronlauge unter Stickstoffatmosphäre. Nach 20 Minuten Rühren gibt man 3,76 g (0,012 Mol) Octadecylbromid zu und erwärmt 12 Stunden auf 80° unter Stickstoff. Dann dampft man im Vakuum zur Trockne ein, säuert mit 2 N Salzsäure an, versetzt mit Chloroform und Wasser, trocknet die organische Phase mit Natriumsulfat und erhält nach Eindampfen die oben genannte Verbindung als farblose Substanz vom Rf-Wert = 0,6 (Dünnschichtplatten Silicagel Merck - Chloroform-Aethanol 9:1), Smp. 100-105° nach Umkristallisieren aus Essigester.

Beispiel 18: Eine vorteilhaft anwendbare Form zur parenteralen, vorzugsweise subcutanen Verabreichung der Lipopeptide erhält man auf folgende Weise:

3 mg Lipopeptid gemäss Beispiel 3 löst man zusammen mit 27 mg Lecithin in einer Mischung aus Chloroform und Methanol 2:1, dampft im Vakuum am Rotationsverdampfer zu einem Lipidfilm ein und versetzt mit 0,2 ml einer sterilen, pyrogenfreien 0,9-prozentigen NaCl-Lösung "Flex-Flac" der Firma VIFOR S.A., Genf. Man beschallt die Probe etwa 2 Minuten bei Raumtemperatur und erhält so eine Suspension von Lecithinkügelchen (Liposomen) von etwa 1 - 5 μ Durchmesser, die das Lipopeptid enthalten. Diese Suspension wird in Dosen von 0,2 ml pro 10 g Maus subcutan oder intraperitoneal verabreicht. Aehnliche pharmazeutische Zubereitungen für die Applikation am Menschen können in ähnlicher Art hergestellt werden.

Beispiel 19: N-Palmitoyl-S-(3,6,9,12,15,18,21,24,27-nonaoxa-octacosanyl)-(L)-cystein:

1 g 1-Brom-(3,6,9,12,15,18,21,24,27-nonoxa)-octacosan (Bromid des Nonaäthylenglykolmonomethyläthers), 0,61 g N-Palmitoyl-L-cystein und 0.6 g Pottasche erwärmt man 15 Stunden in 15 ml Aethanol auf 80° unter Stickstoff. Dann dampft man im Vakuum zur Trockne ein, nimmt den Rückstand in Chloroform auf und schüttelt einmal mit 2N Salzsäure und dreimal mit Wasser aus. Nach Trocknen der $CHCl_3$-Phase erhält man nach Eindampfen 1.2 g der Titelverbindung als Syrup, der über Kieselgel Merck in $CHCl_3$:Methanol 9:1 gereinigt wird. Man erhält nach Vereinigen und Eindampfen der reinen Fraktionen einen farblosen Syrup. Rf = 0.5 $CHCl_3$:Methanol = 8:2 (Dünnschichtplatten, Kieselgel Merck).

Das Bromid des Nonaäthylenglykolmonomethyläthers erhält man aus 10 g Nonaäthylenglykolmonomethyläther 0.87 ml $PBr_3$ und 0.44 ml Pyridin abs. in 6 ml Aether abs. Man mischt die Komponenten bei -10° bis -5° und rührt anschliessend 16 Stunden bei Raumtemperatur. Dann gibt man 30 ml eines Gemisches von Chloroform und Aether (1:1) zu, saugt von Ungelöstem ab und engt das Filtrat im Vakuum zu einem Oel ein. Die Verbindung wird roh in die nächste Stufe eingesetzt.

0,8 g N-Palmitoyl-S-[(3,6,9,12,15,18,21,24,27-nonaoxa)-octacosanyl]-Cys-Ser($Bu^t$)-Ser($Bu^t$)-Phe-Ala-Glu($OBu^t$)$_2$ werden in 10 ml 90%-iger Trifluoressigsäure gelöst und die Lösung nach 40 Minuten bei Raumtemperatur eingedampft. Der Rückstand wird mit Essigester digeriert und über Kaliumhydroxid getrocknet. Man erhält N-Palmitoyl-S-[(3,6,9,12,15,18,21,24,27-nonaoxa)-octacosanyl]-Cys-Ser-Ser-Phe-Ala-Glu-OH als weisses Pulver. Rf-Wert im Dünnschichtchromatogramm auf Silicagel 0.29 (157 c).

Der Ausgangsstoff kann nach der in den Beispielen 1 bis 4 beschriebenen Methodik aus N-Palmitoyl-S-[(3,6,9,12,15,18,21,24,27-nonaoxa)-octacosanyl]-cystein und H-Ser($Bu^t$)-Ser($Bu^t$)-Phe-Ala-Glu($OBu^t$)$_2$ gewonnen werden.

Patentansprüche:
(für alle benannten Länder ausser Oesterreich)

1. Verbindungen der Formel

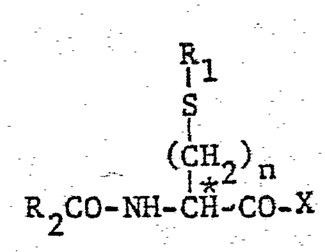

(I)

n = 1,2

* = S/R oder S oder R

worin $R_1$ einen unsubstituierten oder höchstens an einem der dem Schwefelatom nicht benachbarten C-Atome durch eine freie oder eine mit einer einbasischen Carbonsäure veresterte Hydroxygruppe substituierten und gegebenenfalls durch ein oder mehrere Sauerstoffatome in der C-Atom-Kette unterbrochenen, gesättigten oder ungesättigten aliphatischen Kohlenwasserstoffrest mit mindestens 9 C-Atomen, der auch durch maximal 2 cycloaliphatische Kohlenwasserstoffreste mit 5-8 Ring-C-Atomen substituiert sein kann, oder den Rest $-CO-R_1'$, worin $R_1'$ einen gesättigten oder ungesättigten, aliphatischen oder gemischt aliphatisch-cycloaliphatischen, gegebenenfalls auch durch Sauerstoffatome in der C-Atom-Kette unterbrochenen Kohlenwasserstoffrest mit 11-21 C-Atomen, bedeutet, oder 5-Cholesten-3-yl darstellt, $R_2$ einen gesättigten oder ungesättigten, aliphatischen oder gemischt aliphatisch-cycloaliphatischen, gegebenenfalls auch durch Sauerstoffunktionen substituierten Kohlenwasserstoffrest mit 11 - 21 C-Atomen, und X eine peptidisch gebundene Aminosäure mit freier, veresterter oder amidierter Carboxylgruppe, oder eine Aminosäurensequenz von 2 - 10 Aminosäuren, deren terminale Carboxylgruppe in freier, veresterter oder amidierter Form vorliegt, bedeuten, wobei das mit * bezeichnete Asymmetrie-Zentrum die absolute S- oder R-Konfiguration besitzt, Diastereomerengemisch solcher Verbindungen, sowie ihre Salze und Komplexe.

2. Verbindungen gemäss Anspruch 1, worin in Formel (I) $R_1$ einen Rest

$R_3$
|
$CH_2$
|

darstellt, worin $R_3$ einen unsubstituierten oder höchstens durch eine freie oder eine mit einer einbasischen Carbonsäure veresterte Hydroxylgruppe substituierten und gegebenenfalls durch ein oder mehrere Sauerstoffatome in der C-Atom-Kette unterbrochenen, gesättigten oder ungesättigten aliphatischen Kohlenwasserstoffrest mit mindestens 12 C-Atomen, der auch durch maximal 2 cycloaliphatische Kohlenwasserstoffreste mit 5-8 Ring-C-Atomen substituiert sein kann, bedeutet.

3. Verbindungen gemäss einem der Ansprüche 1 oder 2, worin $R_1$ bzw. $R_3$ bzw. ihre durch Sauerstoffunktionen und/oder mit cycloaliphatischen Kohlenwasserstoffresten substituierten Derivate nicht mehr als 60 C-Atome aufweisen, und in denen mindestens eine gerade Kette mit 12-24 C-Atomen vorhanden ist, die gegebenenfalls durch weitere kürzere Ketten substituiert sein kann.

4. Verbindungen gemäss einem der Ansprüche 1-3, worin ein Acylrest $R_2CO$ sich von gesättigten oder ungesättigten Fettsäuren mit 12-22 C-Atomen ableitet.

5. Verbindungen gemäss Anspruch 4, worin die Acylreste sich von der Palmitin-, Stearin-, der Oelsäure, oder Dihydrosterkulsäure ableiten.

6. Verbindungen gemäss einem der Ansprüche 1-5, worin eine Aminosäure X oder die Aminosäuren in einer Aminosäurensequenz X natürliche Aminosäuren der L- oder D-Reihe sind.

7. Verbindungen gemäss Anspruch 6, worin eine Aminosäure ausgewählt ist aus der Gruppe bestehend aus Asparagin-, Glutamin- oder Oxyglutaminsäure, Lysin, Ornithin, Arginin oder Histidin, Asparagin, Glutamin, Serin oder Threonin, Glycin, Alanin, Valin, Norvalin, Leucin, Isoleucin oder Methionin.

8. Verbindungen gemäss Anspruch 7, worin mindestens die Hälfte der Aminosäuren in einer Sequenz X eine hydrophile Gruppe tragen.

9. Verbindungen gemäss einem der Ansprüche 6-13, worin in einer Peptisequenz X sämtliche Aminosäuren mit einer hydrophilenGruppe direkt aneinander gebunden sind und diese Sequenz an die Carboxylgruppe des substituierten Cystein- bzw. Homocysteinteils gebunden ist.

10. Verbindungen gemäss einem der Ansprüche 6-9, worin eine Peptidsequenz X aus 5-Aminosäuren besteht.

11. Verbindungen gemäss einem der Ansprüche 1-10, worin n in Formel 1 des Anspruchs 5 die Zahl 1 bedeutet.

12. Verbindungen gemäss einem der Ansprüche 1-11, worin $R_3$ in Formel I des Anspruchs 2 ein Alkyl der Formel

$$R_4 - \overset{R_6}{\underset{|}{\overset{|}{C}}} - R_5 \qquad (V)$$

bedeutet, worin mindestens einer der Substituenten $R_4$, $R_5$, $R_6$ Alkyl, Alkenyl oder Alkadienyl mit einer geraden Kette von 12-24 C-Atomen darstellt, die gegebenenfalls durch Methyl- oder Aethylgruppen substituiert sein können, und die anderen gegebenenfalls Wasserstoff oder Nieder-alkyl- oder Alkenylreste mit 1-7 C-Atomen sind.

13. Verbindungen gemäss einem der Ansprüche 1-11, worin der Rest $R_1$ gemäss Formel I des Anspruchs 1 folgende Partialformel aufweist

$$\begin{array}{l} R_7 \\ | \\ CH - O - X_1 \\ | \\ CH - O - X_2 \\ | \\ CH_2 \\ | \end{array} \qquad (VI)$$

worin $X_1$ und $X_2$ je Alkyl oder Alkenyl mit 12-20 C-Atomen, $R_7$ Wasserstoff oder Alkyl oder Alkenyl mit 12-20 C-Atomen oder den Rest $-CH_2-O-X_3$ bedeutet, wobei $X_3$ Alkyl oder Alkenyl mit 12-20 C-Atomen bedeuten.

14. Verbindungen gemäss einem der Ansprüche 1-11, worin $R_1$ gemäss Formel I des Anspruchs 5 folgende Partialformel aufweist

$$\begin{array}{l} X_6 \\ | \\ CH - X_5 \\ | \\ CH - O \,.\, CO \,.\, X_4 \\ | \\ CH_2 \\ | \end{array} \qquad (VIII)$$

worin $X_4$ - $X_6$ je Alkyl oder Alkenyl mit 12-20 C-Atomen, $X_6$ aber auch Wasserstoff sein kann.

15. Verbindungen gemäss einem der Ansprüche 1-11, worin $R_1$ den Rest $-CO-R_1'$ darstellt, worin $R_1'$ die gleiche Bedeutung wie $R_3$ in Anspruch 2 besitzt, oder einen Rest gemäss Formel (V) des Anspruchs 11 oder einen solchen gemäss Formel (VI) des Anspruchs 12 oder gemäss Formel (VIII) des Anspruchs 13 besitzt, jedoch in jedem Fall nicht mehr als 21 C-Atome besitzt.

16. Verbindungen gemäss Anspruch 1 der Formel

* = S oder R
oder S/R

** = S oder R
oder S/R

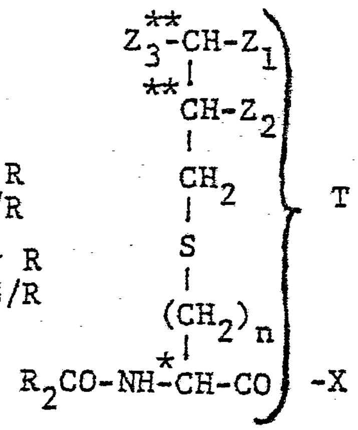

IX

n = 1,2

worin $Z_1$, $Z_2$, $Z_3$ Alkyl oder Alkenyl mit 12-20 C-Atomen, oder $Z_1$ und $Z_2$ die Reste $-O-Z_1'$, bzw. $-O-Z_2'$ bedeuten, wo $Z_1'$ und $Z_2'$ ebenfalls Alkyl oder Alkenyl mit 12-20 C-Atomen darstellen, und $Z_3$ = H oder einen Rest $-CH_2-O-Z_3'$, worin $Z_3'$ Alkyl oder Alkenyl mit 12-20 C-Atomen bedeutet, und X die für Formel (I) gegebene Bedeutung hat, aber im Falle der Aminosäurensequenz 2-5 Aminosäuren aufweist, in welcher vorzugsweise mindestens die Hälfte eine hydrophile Gruppe tragen, und dies auch für den Fall einer einzigen Aminosäure zutrifft, sowie ihre Salze und Komplexe.

17. Verbindungen gemäss Anspruch 1 und 16 der folgenden Formeln

T - Ser - Ser - Asn - Ala - Lys - OH
T - Ser - Ser - Asn - Ala - OH
T - Ser - Ser - Asn - OH
T - Ser - Ser - OH,

sodann die entsprechenden Typen, in welchen Threonin, Glutamin oder Asparagin das Serin ersetzen; ferner die Verbindungstypen der Art:

T - Ser - Ser - Asn - Ala - Glu - OH
T - Ser - Ser - Phe - Ala - Glu - OH
T - Ser - Ser - Phe - Ala - OH
T - Ser - Ser - Phe - OH

und die entsprechenden mit Threonin, Glutamin oder Asparagin als Austauschaminosäuren für Serin, ferner

T - Phe - Phe - Asn - Ala - Lys - OH
T - Glu - Gln - Asn - Ala - Lys - OH
T - Ser - Ser - Asn - Ala - Glu - OH
T - Ser - Ser - Asn - Ala - Ala - OH
T - Val - Lys - Val - Tyr - Pro - OH

T - Phe - Ile - Ile - Phe - Ala - OH
T - Ser - Ser - Phe - Ala - Glu - OH
T - Ser - Thr - Phe - Ala - Glu - OH
T - Ser - Phe - Ala - Glu - OH
T - Thr - Thr - Phe - Ala - Glu - OH
T - Ser - Ser - Asn - Ala - Lys - OH
T - Ser - Ser - Asn - Ala - D - Glu - OH
T - Ser - Ser - D - Asn - Ala - Glu - OH
T - Thr - Thr - Asn - Ala - Lys - OH
T - Ser - Thr - Asn - D - Ala - Lys - OH
T - Ser - Ser - Asn - Ala - Glu - OH
T-D - Ser - Ser - Phe - Ala - D-Glu - OH

und die Amide und Carbonsäureester mit terminaler Carbamid- bzw. Estergruppe, worin T den substituierten Cystein- bzw. Homocystein-Teil gemäss Formel IX des Anspruchs 16 darstellt.

18. Verbindungen gemäss Formel IX des Anspruchs 16, worin $R_2CO$ = Palmitoyl, $Z_1$ und $Z_2$ = Hexadecyloxy, wobei X eine der folgenden Aminosäurensequenzen sein kann

Ser - Ser - Asn - Ala - Lys - OH
Ser - Ser - Asn - Ala - OH
Ser - Ser - Asn - OH
Ser - Ser - OH
Ser - OH

und Verbindungen gemäss Formel IX des Anspruchs 16, worin $R_2CO$ = Stearoyl, $Z_1$ und $Z_2$ = Octadecyloxy, $Z_3$=H, oder $R_2CO$ = Dihydrosterkuloyl, $Z_1$ und $Z_2$ - Hexadecyloxy, $Z_3$ = H, oder $R_2CO$ = Lauroyl, $Z_1$ und $Z_2$ = octadecenyloxy, $Z_3$ = H, oder $R_2CO$ = Lauroyl, $Z_1$ und $Z_2$ = Hexadecyloxy, $Z_3$ = H, bei welchen X eine beliebige der oben für das N-Palmitoyl-Derivat beschriebene Sequenz 2 sein kann, ferner die Verbindungen, die sich aus der Substitution des Serins durch Threonin, Glutaminsäure, Glutamin, Asparaginsäure oder Asparagin und aus der Substitution des Lysins durch Glutaminsäure und Asparagin durch Phenylalanin ergeben.

19. Verbindungen gemäss einem der Ansprüche 1-18, worin sich der Esterrest in der terminalen veresterten Carboxylgruppe von X von einem niederen aliphatischen Alkohol mit 1-7 C-Atomen, einem Aethylenglykol, Propylenglykol oder dem Glyzerin ableitet.

20. Verbindungen gemäss einem der Ansprüche 1-18, worin eine terminale Amidgruppe in X die Amidgruppe $CONH_2$ ist oder eine sich von einem niederen aliphatischen Amin mit 1-7 C-Atomen, dem Pyrrolidin, Piperidin oder Piperazin ableitende Amidgruppe ist.

21. Ein Lipopeptid gemäss Anspruch 1, ausgewählt aus der Gruppe von Verbindungen der Formeln

R-Ser-Ser-D-Asn-Ala-Glu-OH
R-Thr-Thr-Asn-Ala-Lys-OH
R-Ser-Thr-Asn-D-Ala-Lys-OH

R-Ser-Ser-Asn-Ala-Glu-OH
R-Thr-Ser-Phe-Ala-D-Glu-OH
R-Ser-Ser-Phe-Ala-D-Glu-OH
R-Ser-Ser-Phe-Ala-Glu-OH
R-D-Ser-D-Ser-D-Phe-Ala-D-Glu-OH
R-D-Thr-D-Phe-D-Ala-D-Glu-OH

worin R einen gemäss Formel I des Anspruchs 1 substituierten Cystein-Rest von Cysteinen ausgewählt aus der Gruppe bestehend aus

N-palmitoyl-S-[2(R),3-dihexadecyloxypropyl]-(L)-cystein
N-palmitoyl-S-[2(R,S)-hydroxy-octadecyl]-(L)-cystein
N-palmitoyl-S-[2(R,S)-palmitoyloxyoctadecyl]-(L)-cystein
N-palmitoyl-S-octadecyl-(L)-cystein
N-palmitoyl-S-[2(R),3-dihexadecyloxypropyl]-(D,L)-homocystein
N,S-dipalmitoyl-(L)-cystein
N-palmitoyl-S-[2(R,S)-palmitoyloxy-eicosanyl]-(L)-cystein
N-palmitoyl-S-[2(R,S)-hydroxydodecyl]-(L)-cystein
N-palmitoyl-S-[2(R,S)-hydroxyeicosanyl]-(L)-cystein
N-palmitoyl-S-[2(R,S)-palmitoyloxydodecyl]-(L)-cystein
N-palmitoyl-S-[2(R,S)-hydroxy-eicosanyl]-(L)-cystein
N-palmitoyl-S-[2(R,S)-palmitoyloxy-eicosanyl]-(L)-cystein
N-palmitoyl-S-docosanyl-(L)-cystein
N-palmitoyl-S-(3-cholesteryl)-(L)-cystein
N-palmitoyl-S-(3,6-dioxa-docosanyl)-(L)-cystein
N-palmitoyl-S-retinyl-(L)-cystein
N-palmitoyl-S-phythyl-(L)-cystein

bedeutet.

22. Ein Lipopeptid gemäss Anspruch 1, ausgewählt aus der Gruppe

N-palmitoyl-S-[2(R),3-dihexadecyloxypropyl]-Cys-Ser-Ser-Phe-Ala-Glu-OH

N-palmitoyl-S-[2(R,S)-hydroxyoctadecyl]-Cys-Ser-Ser-Phe-Ala-Glu-OH

N-palmitoyl-S-[2(R,S)-palmitoyloxy-octadecyl]-Cys-Ser-Ser-Phe-Ala-Glu-OH

N-palmitoyl-S-octadecyl-Cys-Ser-Ser-Phe-Ala-D-Glu-OH

N-palmitoyl-S-[2(R,S),3-dihexadecyloxypropyl]-(D,L)-homocysteinyl-Ser-Ser-Asn-Ala-Glu-OH

N,S-dipalmitoyl-Cys-Ser-Ser-Phe-Ala-Glu-OH

N,S-dipalmitoyl-Cys-Ser-Ser-Asn-Ala-Pro-$NH_2$

N,S-Dipalmitoyl-Cys-Ser-Ser-Asn-Ala-Val-$OCH_3$

N-palmitoyl-S-octadecyl-D-Cys-Ser-Ser-Phe-Ala-Glu-OH

N-palmitoyl-S-octadecyl-(L,D)-homocysteinyl—Ser-Ser-Phe-Ala-Glu-OH

N-palmitoyl-S-farnesyl-Cys-Ser-Ser-Phe-Ala-Glu-OH

23. Verbindungen der Formel

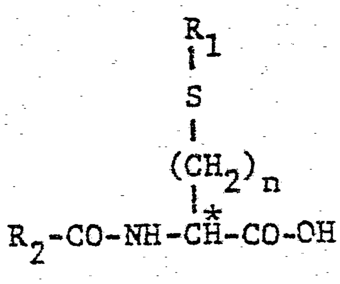

n = 1,2

* = S/R oder S oder R

worin $R_1$ und $R_2$ die für Formel (I) des Anspruchs 1 angegebenen Bedeutungen haben, und entsprechende Verbindungen bzw. Gemische, in denen die Carboxylgruppe in Form eines für die Peptidsynthese geeigneten aktivierten Derivates vorliegt.

24. Verbindungen gemäss Anspruch 23, worin $R_1$ und $R_2$ irgend eine der in den Ansprüchen 2-19 angegebenen Bedeutungen besitzen.

25. Ammoniumsalze, Alkali- oder Erdalkalisalze von sauren Verbindungen, und pharmazeutisch anwendbare, nichttoxische Säureadditionssalze von basischen Verbindungen, und Kupfer-, Zink-, Eisen- oder Kobalt-Komplexsalze von Verbindungen gemäss einem der Ansprüche 1-24.

26. Verbindungen gemäss Anspruch 1, worin jedoch in X mindestens eine der die Aminosäuren substituierenden hydrophilen Gruppen und/oder die terminale Carboxylgruppe in Form einer unter neutralen oder milden sauren Bedingungen abspaltbare Schutzgruppe und/oder die Carboxylgruppe gegebenenfalls in Form eines für die Peptidsynthese geeigneten aktivierten Derivats vorliegt, Gemische von Diastereomeren, und Salze und Komplexe dieser Verbindungen.

27. Verbindungen der Formel

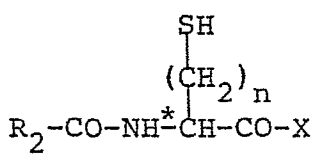

n = 1, 2
* = S/R oder S oder R

worin $R_2$ und X die gleiche Bedeutung wie im Anspruch 1 haben.

28. Pharmazeutische Präparate enthaltend eine Verbindung gemäss einem der Ansprüche 1-25.

29. Eine Verbindung gemäss einem der Ansprüche 1-25 zur Anwendung in einem Verfahren für die therapeutische Behandlung des tierischen oder menschlichen Körpers.

30. Die Verwendung einer Verbindung gemäss einem der Ansprüche 1-25 als immunstimulierendes Mittel, insbesondere als Adjuvantien bei der experimentellen und industriellen Herstellung von Antiseren für Therapie und Diagnostik und bei der Induktion von immunologisch aktivierten Lymphocyten -populationen für Zelltransferverfahren oder als Prophylaktikum oder Therapeutikum gegen Infektionskrankheiten bei Mensch und Tier.

31. Verfahren zur Herstellung von Verbindungen der Formel

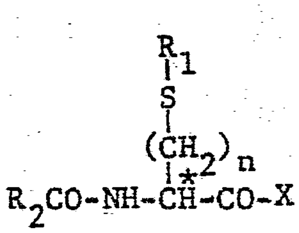

(I)

n = 1,2

* = S/R oder S oder R

worin $R_1$ einen unsubstituierten oder höchstens an einem der dem Schwefelatom nicht benachbarten C-Atome durch eine freie oder eine mit einer einbasischen Carbonsäure veresterte Hydroxygruppe substituierten und gegebenenfalls durch ein- oder mehrere Sauerstoffatome in der C-Atom-Kette unterbrochenen, gesättigten oder ungesättigten aliphatischen Kohlenwasserstoffrest mit mindestens 9 C-Atomen, der auch durch maximal 2 cycloaliphatische Kohlenwasserstoffreste mit 5-8 Ring-C-Atomen substituiert sein kann, oder den Rest $-CO-R_1'$, worin $R_1'$ einen gesättigten oder ungesättigten, aliphatischen oder gemischt aliphatisch-cycloaliphatischen, gegebenenfalls auch durch Sauerstoffatome in der C-Atom-Kette unterbrochenen Kohlenwasserstoffrest mit 11-21 C-Atomen, bedeutet, oder 5-Cholesten-3-yl darstellt, $R_2$ einen gesättigten oder ungesättigten, aliphatischen oder gemischt aliphatisch-cycloaliphatischen, gegebenenfalls auch durch Sauerstoffunktionen substituierten Kohlenwasserstoffrest mit 11 - 21 C-Atomen, und X eine peptidisch gebundene Aminosäure mit freier, veresterter oder amidierter Carboxylgruppe, oder eine Aminosäurensequenz von 2 - 10 Aminosäuren, deren terminale Carboxylgruppe in freier, veresterter oder amidierter Form vorliegt, bedeuten, wobei das mit * bezeichnete Asymmetrie-Zentrum die absolute S- oder R- oder S/R-Konfiguration besitzt oder Gemische von Diastereomeren dieser Verbindungen, dadurch gekennzeichnet, dass man

a) in einer Verbindung der Formel

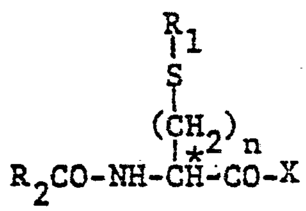

(X)

n = 1,2

* = S/R oder S oder R

worin $R_1$ und $R_2$ die für Formel (I) angegebene Bedeutung haben und Y eine X in der Formel (I) entsprechende Aminosäure oder Aminosäurensequenz darstellt, worin jedoch mindestens eine der die Aminosäuren substituierenden hydrophilen Gruppen und/oder die terminale Carboxylgruppe durch eine unter neutralen oder milden sauren Bedingungen abspaltbare Schutzgruppe geschützt ist, oder in einem Salz einer solchen Verbindung, die Schutzgruppe(n) abspaltet, oder

b) dass man eine Verbindung der Formel

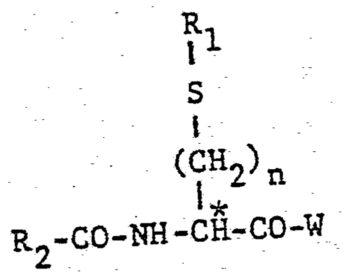

(XI)

n = 1,2

* = S/R oder S
oder R

worin $R_1$ und $R_2$ die für Formel (I) angegebene Bedeutung haben und W = OH oder eine Aminosäure oder eine unvollständige Sequenz von Aminosäuren gemäss X in Formel (I) darstellt, mit einer Verbindung der Formel

$$NH_2 - Z_1 \qquad (XII)$$

wo $Z_1$ eine der Gruppe X in Formel (I) entsprechende Gruppe bzw. eine zur genannten Aminosäure oder unvollständigen Sequenz von Aminosäuren gemäss X komplementären Aminosäuresequenz bzw. Aminosäure bedeutet, in welchen Sequenzen jedoch keine freien Aminogruppen vorhanden sind, oder einem Salz derselben, umsetzt, oder

c) in einer Verbindung entsprechend derjenigen der Formel I, in welcher im Rest $R_1$ mindestens eine freie Hydroxygruppe vorhanden ist, und im Rest X aber keine freien Hydroxygruppen vorhanden sind, die freien Hydroxygruppen veräthert, oder

d) in einer Verbindung gemäss Formel (I), worin der Rest $R_1$ eine freie Hydroxygruppe aufweist, aber keine freien Hydroxylgruppen in X vorhanden sind, die freie Hydroxygruppe mittels eines acylierenden Mittels verestert, oder

e) eine Verbindung der Formel

```
          SH
          |
         (CH2)n
          |*
R2CO-NH-CH-CO-X
```

$n = 1,2$ (XIII) ,
* = S/R oder S oder R

worin $R_2$ und X die oben angegebene Bedeutung haben, aber keine freien Hydroxygruppen in X vorhanden sind, mit einem acylierenden Mittel behandelt, oder

f) eine Verbindung der Formel

```
          SH
          |
         (CH2)
          |*
R2CO-NH-CH-CO-X
```

$n = 1,2$ (XIV),
* = S/R oder S oder R

worin $R_2$ und X die gleiche Bedeutung wie in Formel I haben, mit einer Verbindung der Formel

$R_1 - T$ (XV),

worin T eine reaktionsfähige funktionell abgewandelte Hydroxygruppe bedeutet, in Gegenwart eines basischen Mittels umsetzt, oder

g) eine Verbindung der Formel

```
      R1
      |
      S
      |
     (CH2)n
      |*
H2N-CH-CO-X
```

$n = 1,2$
* = S/R oder S oder R (XVI),

worin X und $R_1$ die gleiche Bedeutung wie in Formel I haben, jedoch keine Hydroxylgruppen und Aminogruppen in X vorhanden sind, mit einem Acylierungsmittel behandelt,

und, wenn erwünscht, in erhaltenen Verbindungen mit freier terminaler Carboxylgruppe dieselbe in eine Amidgruppe oder Estergruppe überführt, und/oder die Verbindungen in ihre Salze oder Komplexe überführt.

32. Verbindungen gemäss einem der Ansprüche 1-20 und 23-27, worin die Konfiguration am C*-Atom des Cystein-Restes R ist.

Patentansprüche: (für Oesterreich)

1. Verfahren zur Herstellung von Verbindungen der Formel

$$\begin{array}{l} \quad\quad\quad R_1 \\ \quad\quad\quad | \\ \quad\quad\quad S \\ \quad\quad\quad | \\ \quad\quad (CH_2)_n \\ \quad\quad\quad |\,* \\ R_2CO-NH-CH-CO-X \end{array} \qquad \begin{array}{l} n = 1,2 \\ * = S/R \text{ oder } S \text{ oder } R \end{array} \qquad (I)$$

worin $R_1$ einen unsubstituierten oder höchstens an einem der dem Schwefelatom nicht benachbarten C-Atome durch eine freie oder eine mit einer einbasischen Carbonsäure veresterte Hydroxygruppe substituierten und gegebenenfalls durch ein- oder mehrere Sauerstoffatome in der C-Atom-Kette unterbrochenen, gesättigten oder ungesättigten aliphatischen Kohlenwasserstoffrest mit mindestens 9 C-Atomen, der auch durch maximal 2 cycloaliphatische Kohlenwasserstoffreste mit 5-8 Ring-C-Atomen substituiert sein kann, oder den Rest $-CO-R_1'$, worin $R_1'$ einen gesättigten oder ungesättigten, aliphatischen oder gemischt aliphatisch-cycloaliphatischen, gegebenenfalls auch durch Sauerstoffatome in der C-Atom-Kette unterbrochenen Kohlenwasserstoffrest mit 11-21 C-Atomen, bedeutet, oder 5-Cholesten-3-yl darstellt, $R_2$ einen gesättigten oder ungesättigten, aliphatischen oder gemischt aliphatisch-cycloaliphatischen, gegebenenfalls auch durch Sauerstoffunktionen substituierten Kohlenwasserstoffrest mit 11 - 21 C-Atomen, und X eine peptidisch gebundene Aminosäure mit freier, veresterter oder amidierter Carboxylgruppe, oder eine Aminosäurensequenz von 2 - 10 Aminosäuren, deren terminale Carboxylgruppe in freier, veresterter oder amidierter Form vorliegt, bedeuten, wobei das mit * bezeichnete Asymmetrie-Zentrum die

absolute S- oder R-Konfiguration besitzt, dadurch gekennzeichnet, dass man

a) in einer Verbindung der Formel

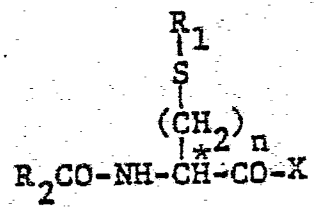

(X)

n = 1,2

* = S/R oder S oder R

worin $R_1$ und $R_2$ die für Formel (I) angegebene Bedeutung haben und Y eine X in der Formel (I) entsprechende Aminosäure oder Aminosäurensequenz darstellt, worin jedoch mindestens eine der die Aminosäuren substituierenden hydrophilen Gruppen und/oder die terminale Carboxylgruppe durch eine unter neutralen oder milden sauren Bedingungen abspaltbare Schutzgruppe geschützt ist, oder in einem Salz einer solchen Verbindung, die Schutzgruppe(n) abspaltet, oder

b) dass man eine Verbindung der Formel

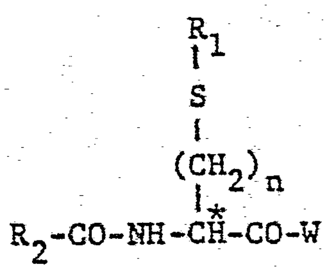

(XI)

n = 1,2

* = S/R oder S oder R

worin $R_1$ und $R_2$ die für Formel (I) angegebene Bedeutung haben und W = OH oder eine Aminosäure oder eine unvollständige Sequenz von Aminosäuren gemäss X in Formel (I)

darstellt, mit einer Verbindung der Formel

$$NH_2 - Z_1 \qquad (XII)$$

wo $Z_1$ eine der Gruppe X in Formel (I) entsprechende Gruppe bzw. eine zur genannten Aminosäure oder unvollständigen Sequenz von Aminosäuren gemäss X komplementären Aminosäuresequenz bzw. Aminosäure bedeutet, in welchen Sequenzen jedoch keine freien Aminogruppen vorhanden sind, oder einem Salz derselben, umsetzt, oder

c) in einer Verbindung entsprechend derjenigen der Formel I, in welcher im Rest $R_1$ mindestens eine freie Hydroxygruppe vorhanden ist, und im Rest X aber keine freien Hydroxygruppen vorhanden sind, die freien Hydroxygruppen mit Diazomethan veräthert, oder

d) in einer Verbindung gemäss Formel (I), worin der Rest $R_1$ eine freie Hydroxygruppe aufweist, aber keine freien Hydroxylgruppen in X vorhanden sind, die freie Hydroxygruppe mittels eines acylierenden Mittels verestert, oder

e) eine Verbindung der Formel

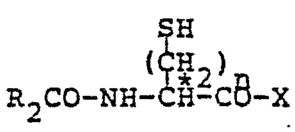

n = 1,2
* = S/R oder S oder R

(XIII) ,

worin $R_2$ und X die oben angegebene Bedeutung haben, aber keine freien Hydroxygruppen in X vorhanden sind, mit einem acylierenden Mittel behandelt, oder

f) eine Verbindung der Formel

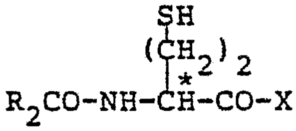

n = 1,2
* = S/R oder S oder R

(XIV),

worin $R_2$ und X die gleiche Bedeutung wie in Formel I haben, mit einer Verbindung der Formel

$$R_1 - T \qquad (XV),$$

worin T eine reaktionsfähige funktionell abgewandelte Hydroxygruppe bedeutet, in Gegenwart eines basischen Mittels umsetzt, oder

g) eine Verbindung der Formel

$$\begin{array}{c} R_1 \\ | \\ S \\ | \\ (CH_2)_n \\ |{*} \\ H_2N-CH-CO-X \end{array} \qquad \begin{array}{l} n = 1,2 \\ * = S/R \text{ oder } S \text{ oder } R \end{array} \qquad (XVI),$$

worin X und $R_1$ die gleiche Bedeutung wie in Formel I haben, jedoch keine Hydroxylgruppen und Aminogruppen in X vorhanden sind, mit einem Acylierungsmittel behandelt,

und, wenn erwünscht, in erhaltenen Verbindungen mit freier terminaler Carboxylgruppe dieselbe in eine Amidgruppe oder Estergruppe überführt, und/oder die Verbindungen in ihre Salze oder Komplexe überführt.

2. Verfahren gemäss Anspruch 1, dadurch gekennzeichnet, dass man Verbindungen herstellt, worin in Formel (I) $R_1$ einen Rest

$$\begin{array}{c} R_3 \\ | \\ CH_2 \\ | \end{array}$$

darstellt, worin $R_3$ einen unsubstituierten oder höchstens durch eine freie oder eine mit einer einbasischen Carbonsäure veresterte Hydroxylgruppe substituierten und gegebenenfalls durch ein oder mehrere Sauerstoffatome in der C-Atom-Kette unterbrochenen, gesättigten oder ungesättigten aliphatischen Kohlenwasserstoffrest mit mindestens 12 C-Atomen, der auch durch maximal 2 cycloaliphatische Kohlenwasserstoffreste mit 5-8 Ring-C-Atomen substituiert sein kann, bedeutet.

3. Verfahren gemäss einem der Ansprüche 1 oder 2, dadurch gekennzeichnet, dass man Verbindungen herstellt, worin $R_1$ bzw. $R_3$ bzw. ihre durch Sauerstoffunktionen und/oder mit cycloaliphatischen Kohlenwasserstoffresten substituierten Derivate nicht mehr als 60 C-Atome aufweisen, und in denen mindestens eine gerade Kette mit 12-24 C-Atomen vorhanden ist, die gegebenenfalls durch weitere kürzere Ketten substituiert sein kann.

4. Verfahren gemäss einem der Ansprüche 1-3, worin ein Acylrest $R_2CO$ sich von gesättigten oder ungesättigten Fettsäuren mit 12-22 C-Atomen ableitet.

5. Verfahren gemäss Anspruch 4, worin die Acylreste sich von der Palmitin-, Stearin-, der Oelsäure, oder Dihydrosterkulsäure ableiten.

6. Verfahren gemäss einem der Ansprüche 1-4, worin eine Aminosäure X oder die Aminosäuren in einer Aminosäurensequenz X natürliche Aminosäuren der L- oder D-Reihe sind.

7. Verfahren gemäss Anspruch 6, worin eine Aminosäure ausgewählt ist aus der Gruppe bestehend aus Asparagin-, Glutamin- oder Oxyglutaminsäure, Lysin, Ornithin, Arginin oder Histidin, Asparagin, Glutamin, Serin oder Threonin, Glycin, Alanin, Valin, Norvalin, Leucin, Isoleucin oder Methionin.

8. Verfahren gemäss Anspruch 7, worin mindestens die Hälfte der Aminosäuren in einer Sequenz X eine hydrophile Gruppe tragen.

9. Verfahren gemäss einem der Ansprüche 6-13, dadurch gekennzeichnet, dass man Verbindungen herstellt, worin in einer Peptisequenz X sämtliche Aminosäuren mit einer hydrophilen Gruppe direkt aneinander gebunden sind und diese Sequenz an die Carboxylgruppe des substituierten Cystein- bzw. Homocysteinteils gebunden ist.

10. Verfahren gemäss einem der Ansprüche 6-9, dadurch gekennzeichnet, dass man Verbindungen herstellt, worin eine Peptidsequenz X aus 5-Aminosäuren besteht.

11. Verfahren gemäss einem der Ansprüche 1-10, dadurch gekennzeichnet, dass man Verbindungen herstellt, worin n in Formel 1 des Anspruchs 5 die Zahl 1 bedeutet.

12. Verfahren gemäss einem der Ansprüche 1-11, dadurch gekennzeichnet, dass man Verbindungen herstellt, worin $R_3$ in Formel I des Anspruchs 6 ein Alkyl der Formel

$$R_4 - \overset{\displaystyle R_6}{\overset{|}{\underset{|}{C}}} - R_5 \qquad (V)$$

bedeutet, worin mindestens einer der Substituenten $R_4$, $R_5$, $R_6$ Alkyl, Alkenyl oder Alkadienyl mit einer geraden Kette von 12-24 C-Atomen darstellt, die gegebenenfalls durch Methyl- oder Aethylgruppen substituiert sein können, und die anderen gegebenenfalls Wasserstoff oder Nieder-alkyl- oder Alkenylreste mit 1-7 C-Atomen sind.

13. Verfahren gemäss einem der Ansprüche 1-11, dadurch gekennzeichnet, dass man Verbindungen herstellt, worin der Rest $R_1$ gemäss Formel I des Anspruchs 1 folgende Partialformel aufweist

$R_7$
|
$CH - O - X_1$
|
$CH - O - X_2$ (VI)
|
$CH_2$
|

worin $X_1$ und $X_2$ je Alkyl oder Alkenyl mit 12-20 C-Atomen, $R_7$ Wasserstoff oder Alkyl oder Alkenyl mit 12-20 C-Atomen oder den Rest $-CH_2-O-X_3$ bedeutet, wobei $X_3$ Alkyl oder Alkenyl mit 12-20 C-Atomen bedeuten.

14. Verfahren gemäss einem der Ansprüche 1-11, dadurch gekennzeichnet, dass man Verbindungen herstellt, worin $R_1$ gemäss Formel I des Anspruchs 5 folgende Partialformel aufweist

$X_6$
|
$CH - X_5$
|
$CH - O . CO . X_4$ (VIII)
|
$CH_2$
|

worin $X_4$ - $X_6$ je Alkyl oder Alkenyl mit 12-20 C-Atomen, $X_6$ aber auch Wasserstoff sein kann.

15. Verfahren nach einem der Ansprüche 1-11, dadurch gekennzeichnet, dass man Verbindungen herstellt, worin $R_1$ den Rest $-CO-R_1'$ darstellt, worin $R_1'$ die gleiche Bedeutung wie $R_3$ in Anspruch 2 besitzt, oder einen Rest gemäss Formel (V) des Anspruchs 11 oder einen solchen gemäss Formel (VI) des Anspruchs 12 oder gemäss Formel (VIII) des Anspruchs 13 besitzt, jedoch in jedem Fall nicht mehr als 21 C-Atome besitzt.

16. Verfahren gemäss Anspruch 1, dadurch gekennzeichnet, dass man Verbindungen der Formel

* = S oder R
oder S/R

** = S oder R
oder S/R

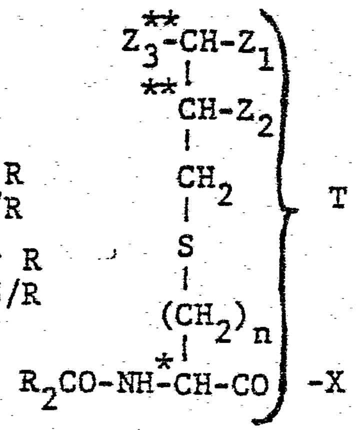

IX

n = 1,2

worin $Z_1$, $Z_2$, $Z_3$ Alkyl oder Alkenyl mit 12-20 C-Atomen, oder $Z_1$ und $Z_2$ die Reste $-O-Z_1'$, bzw. $-O-Z_2'$ bedeuten, wo $Z_1'$ und $Z_2'$ ebenfalls Alkyl oder Alkenyl mit 12-20 C-Atomen darstellen, und $Z_3$ = H oder einen Rest $-CH_2-O-Z_3'$, worin $Z_3'$ Alkyl oder Alkenyl mit 12-20 C-Atomen bedeutet, und X die für Formel (I) gegebene Bedeutung hat, aber im Falle der Aminosäurensequenz 2-5 Aminosäuren aufweist, in welcher vorzugsweise mindestens die Hälfte eine hydrophile Gruppe tragen, und dies auch für den Fall einer einzigen Aminosäure zutrifft, sowie ihre Salze und Komplexe herstellt.

17. Verfahren gemäss Anspruch 1 und 16, dadurch gekennzeichnet, dass man Verbindungen der folgenden Formeln herstellt

T - Ser - Ser - Asn - Ala - Lys - OH
T - Ser - Ser - Asn - Ala - OH
T - Ser - Ser - Asn - OH
T - Ser - Ser - OH,

sodann die entsprechenden Typen, in welchen Threonin, Glutamin oder Asparagin das Serin ersetzen; ferner die Verbindungstypen der Art:

T - Ser - Ser - Asn - Ala - Glu - OH
T - Ser - Ser - Phe - Ala - Glu - OH
T - Ser - Ser - Phe - Ala - OH
T - Ser - Ser - Phe - OH

und die entsprechenden mit Threonin, Glutamin oder Asparagin als Austauschaminosäuren für Serin, ferner

T - Phe - Phe - Asn - Ala - Lys - OH
T - Glu - Gln - Asn - Ala - Lys - OH
T - Ser - Ser - Asn - Ala - Glu - OH
T - Ser - Ser - Asn - Ala - Ala - OH
T - Val - Lys - Val - Tyr - Pro - OH
T - Phe - Ile - Ile - Phe - Ala - OH
T - Ser - Ser - Phe - Ala - Glu - OH
T - Ser - Thr - Phe - Ala - Glu - OH
T - Ser - Phe - Ala - Glu - OH
T - Thr - Thr - Phe - Ala - Glu - OH
T - Ser - Ser - Asn - Ala - Lys - OH
T - Ser - Ser - Asn - Ala - D - Glu - OH
T - Ser - Ser - D - Asn - Ala - Glu - OH
T - Thr - Thr - Asn - Ala - Lys - OH
T - Ser - Thr - Asn - D - Ala - Lys - OH
T - Ser - Ser - Asn - Ala - Glu - OH
T-D - Ser - Ser - Phe - Ala - D-Glu - OH

und die Amide und Carbonsäureester mit terminaler Carbamid- bzw. Estergruppe, worin T den substituierten Cystein- bzw. Homocystein-Teil gemäss Formel IX des Anspruchs 16 darstellt.

18. Verfahren gemäss Anspruch 16, dadurch gekennzeichnet, dass man Verbindungen der Formel IX herstellt, worin $R_2CO$ = Palmitoyl, $Z_1$ und $Z_2$ = Hexadecyloxy, wobei X eine der folgenden Aminosäurensequenzen sein kann

Ser - Ser - Asn - Ala - Lys - OH
Ser - Ser - Asn - Ala - OH
Ser - Ser - Asn - OH
Ser - Ser - OH
Ser - OH

und Verbindungen gemäss Formel IX des Anspruchs 16, worin $R_2CO$ = Stearoyl, $Z_1$ und $Z_2$ = Octadecyloxy, $Z_3$=H, oder $R_2CO$ = Dihydrosterkuloyl, $Z_1$ und $Z_2$ - Hexadecyloxy, $Z_3$ = H, oder $R_2CO$ = Lauroyl, $Z_1$ und $Z_2$ = octadecenyloxy, $Z_3$ = H, oder $R_2CO$ = Lauroyl, $Z_1$ und $Z_2$ = Hexadecyloxy, $Z_3$ = H, bei welchen X eine beliebige der oben für das N-Palmitoyl-Derivat beschriebene Sequenz 2 sein kann, ferner die Verbindungen, die sich aus der Substitution des Serins durch Threonin, Glutaminsäure, Glutamin, Asparaginsäure oder Asparagin und aus der Substitution des Lysins durch Glutaminsäure und Asparagin durch Phenylalanin ergeben.

19. Verfahren gemäss einem der Ansprüche 1-18, dadurch gekennzeichnet, dass man Verbindungen herstellt, worin sich der Esterrest in der terminalen veresterten Carboxylgruppe von X von einem niederen aliphatischen Alkohol mit 1-7 C-Atomen, einem Aethylenglykol, Propylenglykol oder dem Glyzerin ableitet.

20. Verfahren gemäss einem der Ansprüche 1-18, dadurch gekennzeichnet, dass man Verbindungen herstellt, worin eine terminale Amidgruppe in X die Amidgruppe $CONH_2$ ist oder eine sich von einem niederen aliphatischen Amin mit 1-7 C-Atomen, dem Pyrrolidin, Piperidin oder Piperazin ableitende Amidgruppe ist.

21. Verfahren gemäss Anspruch 1, dadurch gekennzeichnet, dass man Verbindungen der folgenden Formeln herstellt

R-Ser-Ser-D-Asn-Ala-Glu-OH
R-Thr-Thr-Asn-Ala-Lys-OH
R-Ser-Thr-Asn-D-Ala-Lys-OH
R-Ser-Ser-Asn-Ala-Glu-OH
R-Thr-Ser-Phe-Ala-D-Glu-OH
R-Ser-Ser-Phe-Ala-D-Glu-OH
R-Ser-Ser-Phe-Ala-Glu-OH
R-D-Ser-D-Ser-D-Phe-Ala-D-Glu-OH
R-D-Thr-D-Phe-D-Ala-D-Glu-OH

worin R einen gemäss Formel I des Anspruchs 1 substituierten Cystein-Rest von Cysteinen ausgewählt aus der Gruppe bestehend aus
N-palmitoyl-S-[2(R),3-dihexadecyloxypropyl]-(L)-cystein
N-palmitoyl-S-[2(R,S)-hydroxy-octadecyl]-(L)-cystein
N-palmitoyl-S-[2(R,S)-palmitoyloxyoctadecyl]-(L)-cystein
N-palmitoyl-S-octadecyl-(L)-cystein
N-palmitoyl-S-[2(R),3-dihexadecyloxypropyl]-(D,L)-homocystein
N,S-dipalmitoyl-(L)-cystein
N-palmitoyl-S-[2(R,S)-palmitoyloxy-eicosanyl]-(L)-cystein
N-palmitoyl-S-[2(R,S)-hydroxydodecyl]-(L)-cystein
N-palmitoyl-S-[2(R,S)-hydroxyeicosanyl]-(L)-cystein
N-palmitoyl-S-[2(R,S)-palmitoyloxydodecyl]-(L)-cystein
N-palmitoyl-S-[2(R,S)-hydroxy-eicosanyl]-(L)-cystein
N-palmitoyl-S-[2(R,S)-palmitoyloxy-eicosanyl]-(L)-cystein
N-palmitoyl-S-docosanyl-(L)-cystein
N-palmitoyl-S-(3-cholesteryl)-(L)-cystein
N-palmitoyl-S-(3,6-dioxa-docosanyl)-(L)-cystein
N-palmitoyl-S-retinyl-(L)-cystein
N-palmitoyl-S-phythyl-(L)-cystein

bedeutet.

22. Verfahren gemäss Anspruch 1, dadurch gekennzeichnet, dass man ein Lipopeptid herstellt, das aus folgender Gruppe ausgewählt ist:

N-palmitoyl-S-[2(R),3-dihexadecyloxypropyl]-Cys-Ser-Ser-Phe-Ala-Glu-OH
N-palmitoyl-S-[2(R,S)-hydroxyoctadecyl]-Cys-Ser-Ser-Phe-Ala-Glu-OH
N-palmitoyl-S-[2(R,S)-palmitoyloxy-octadecyl]-Cys-Ser-Ser-Phe-Ala-Glu-OH
N-palmitoyl-S-octadecyl-Cys-Ser-Ser-Phe-Ala-D-Glu-OH
N-palmitoyl-S-[2(R,S),3-dihexadecyloxypropyl]-(D,L)-homocysteinyl-Ser-Ser-Asn-Ala-Glu-OH
N,S-dipalmitoyl-Cys-Ser-Ser-Phe-Ala-Glu-OH
N,S-dipalmitoyl-Cys-Ser-Ser-Asn-Ala-Pro-$NH_2$
N,S-Dipalmitoyl-Cys-Ser-Ser-Asn-Ala-Val-$OCH_3$
N-palmitoyl-S-octadecyl-D-Cys-Ser-Ser-Phe-Ala-Glu-OH
N-palmitoyl-S-octadecyl-(L,D)-homocysteinyl—Ser-Ser-Phe-Ala-Glu-OH
N-palmitoyl-S-farnesyl-Cys-Ser-Ser-Phe-Ala-Glu-OH.

23. Verfahren zur Herstellung von Verbindungen der Formel

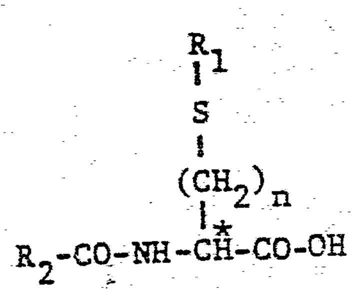

n = 1,2
* = S/R oder S
oder R

worin $R_1$ und $R_2$ die für Formel (1) des Anspruches 1 angegebenen Bedeutungen haben, und entsprechende Verbindungen bzw. Gemische, in denen die Carboxylgruppe in Form eines für die Peptidsynthese geeigneten aktivierten Derivates vorliegt, dadurch gekennzeichnet, dass man ein substituiertes Cystein oder Homocystein der Formel

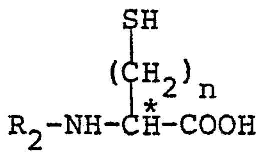

n = 1, 2     * =S/R oder S oder R

worin $R_2$ die gleiche Bedeutung wie in Formel I hat, in Gegenwart eines basischen Mittels mit einer Verbindung der Formel

$$R_1 - T$$

worin T eine reaktionsfähige, funktionell abgewandelte Hydroxygruppe bedeutet, umsetzt.

24. Verfahren gemäss Anspruch 23, dadurch gekennzeichnet, dass man Verbindungen herstellt, worin $R_1$ und $R_2$ irgend eine der in den Ansprüchen 2-19 angegebenen Bedeutungen haben.

25. Verfahren gemäss einem der Ansprüche 1-24, dadurch gekennzeichnet, dass man Ammoniumsalze, Alkali- oder Erdalkalisalze von sauren Verbindungen, und pharmazeutisch anwendbare, nicht toxische Säureadditionssalze von basischen Verbindungen, und Kupfer-, Zink-, Eisen- oder Kobalt-Komplexsalze der Verbindungen herstellt.

26. Verfahren gemäss Anspruch 1b) bis 1g), dadurch gekennzeichnet, dass man Ausgangsstoffe verwendet, in denen X gemäss Formel (I) oder gemäss den Formeln XIII-XVI mindestens eine geschützte hydrophile Gruppe und/oder die terminale Carboxylgruppe in geschützter Form enthält, oder eine Gruppe OH anstelle von W gegebenenfalls auch in geschützter Form vorliegt.

27. Verfahren gemäss einem der Ansprüche 1-26, dadurch gekennzeichnet, dass man die in den Beispielen beschriebenen Verbindungen herstellt.

28. Verfahren zur Herstellung eines pharmazeutischen Präparates, dadurch gekennzeichnet, dass man eines der in den Ansprüchen 1-27 genannten Verfahrensprodukte mit einem pharmazeutischen Trägermaterial verarbeitet.

29. Verfahren nach einem der Ansprüche 1-28, dadurch gekennzeichnet, dass man Verbindungen herstellt, die am C*-Atom des Cystein-Restes die R-Konfiguration haben.